# EUROPEAN PATENT APPLICATION

(11) **EP 3 441 761 A1**
(43) Date of publication of application: **13.02.2019**
(21) Application number: 17778902.1
(22) Date of filing: 01.03.2017
(51) Int. Cl.: G01N 33/48, G01N 33/53, G01N 33/536

(54) **FLUORESCENT IMMUNOSTAINING METHOD**

(30) Priority: 06.04.2016 JP 2016076433
(71) Applicant: Konica Minolta, Inc., Tokyo 100-7015 (JP)
(72) Inventor: TAKAHASHI Masaru, Tokyo 100-7015 (JP); MOTOKUI Yasuyuki, Tokyo 100-7015 (JP)
(74) Representative: Hoffmann Eitle
(86) International application number: PCT/JP2017/008064
(87) International publication number: WO 2017/175523

(57) **Abstract**

A fluorescent immunostaining method including a first reaction step of specifically binding and fixing primary antibodies 1a, 1b to an antigen P as a staining subject and a second reaction step of specifically binding a secondary antibody 2a that has been modified by fluorescent nanoparticle 5 or is to be subsequently modified thereby, to the bound and fixed primary antibodies 1a, 1b in which plural secondary antibodies 2a are fixed to the antigen P by way of the primary antibodies 1a, 1b, making it possible to detect lowly-expressed antigens.

## Description

### Technical Field

The present invention relates to a fluorescent immunostaining method which allows obtainment of a higher fluorescence signal than before.

### Background Art

To obtain data for pathological diagnosis, conventionally a fluorescent immunostaining method is carried out in which a sample slide is prepared from a biopsy sample of body tissues of a subject who is suspected of having a disorder and the presence of an antigen derived from the disorder (specific antigen) in the sample slide is examined by using the specific antibody.

For example, a method of determining the presence or absence of a specific antigen using fluorescence, in which a primary antibody and a fluorescence labeled secondary antibody are allowed to bind in the order against a specific antigen in a sample slide, is carried out (for example, Patent Literature 1).

In Patent Literature 1, as a fluorescent immunostaining method, a method of determining the presence or absence of a specific antigen, in which anti HER2 rabbit monoclonal antibody (4B5) (primary antibody) is bound to HER2 (specific antigen) of a sample slide, biotin-labeled anti rabbit antibody (secondary antibody) is bound to the primary antibody, and then the SA part of a fluorescent dye-containing nanoparticle (fluorescent nanoparticle)-streptavidin (SA) complex is bound to the biotin of the secondary antibody, is disclosed.

Herein, like the techniques of a related art that are described above (Patent Literature 1), a monoclonal antibody is generally used as a primary antibody and a secondary antibody.

### Citation List

### Patent Literature

Patent Literature 1: JP 2015-059806 A

### Summary of Invention

### Technical Problem

However, use of a monoclonal antibody as a primary antibody and a secondary antibody has a problem that one antigen is bound and fixed with only one primary antibody and only one fluorescent nanoparticle, respectively, so that it is difficult to detect lowly-expressed antigens.

The present invention is devised in consideration of the above problems, and object of the present invention is to provide a fluorescent immunostaining method which allows detection of lowly-expressed antigens.

### Solution to Problem

The inventors of the present invention found that the aforementioned problems can be solved by sensitization based on fixing of two or more secondary antibodies that are fluorescence labeled (or to be subsequently fluorescence labeled) with a fluorescent nanoparticle, to one antigen molecule, by way of a primary antibody, and the present invention is completed accordingly.

Namely, the present invention provides a fluorescent immunostaining method as follows.
[1] A fluorescent immunostaining method including: a first reaction step of specifically binding and fixing a primary antibody to an antigen as a staining subject; a second reaction step of specifically binding a secondary antibody to the bound and fixed primary antibody; and a third reaction step of fluorescence labeling the secondary antibody with a fluorescent nanoparticle,
   wherein a plurality of secondary antibodies are fixed to one molecule of the antigen by way of the primary antibody.
[2] The fluorescent immunostaining method according to [1], wherein the primary antibody is a free primary antibody and the secondary antibody is a free secondary antibody. Herein, the expression "free primary antibody" means a primary antibody which is not fixed to a support or the like and exists in a free state. Furthermore, the expression "free secondary antibody" means a secondary antibody which is not fixed to a support or the like and exists in a free state.
[3] The fluorescent immunostaining method according to [1], wherein the primary antibody is a primary antibody fixed on a surface of a non-fluorescent nanoparticle not containing fluorescent material and the secondary antibody is a free secondary antibody.
[4] The fluorescent immunostaining method according to [1], wherein the primary antibody is a free primary antibody and the secondary antibody is a secondary antibody fixed on a surface of a fluorescent nanoparticle.
[5] The fluorescent immunostaining method according to [1], wherein the primary antibody is a primary antibody fixed on a surface of a non-fluorescent nanoparticle and the secondary antibody is a secondary antibody fixed on a surface of a fluorescent nanoparticle.
[6] The fluorescent immunostaining method according to any one of [1] to [5], wherein the primary antibody is a polyclonal antibody and the secondary antibody is a monoclonal antibody.
[7] The fluorescent immunostaining method according to any one of [1] to [5], wherein the primary antibody is a monoclonal antibody and the secondary antibody is a polyclonal antibody.
[8] The fluorescent immunostaining method according to any one of [1] to [5], wherein the primary antibody is a polyclonal antibody and the secondary antibody is a polyclonal antibody.
[9] The fluorescent immunostaining method according to any one of [1] and [3] to [5], wherein the primary antibody is a monoclonal antibody and the secondary antibody is a monoclonal antibody.
[10] The fluorescent immunostaining method according to [1], wherein
   a second binding group material is added to the secondary antibody, a first binding group material is added to the fluorescent nanoparticle, and
   after the second reaction step, according to binding between the secondary antibody and fluorescent nanoparticle by way of specific binding between the first binding group material and second binding group material, the fluorescence labeling is achieved.
[11] The fluorescent immunostaining method according to [10], wherein combination of the first binding group material and second binding group material is biotin/avidin, or hapten/anti hapten antibody.

### Advantageous Effects of Invention

According to the present invention, a fluorescent immunostaining method allowing detection of lowly-expressed antigens is provided so that antigens can be detected at higher sensitivity compared to the techniques of a related art. In addition, higher accuracy of protein quantification property can be obtained.

### Brief Description of Drawings

Each of Figs. 1(A) to 1(C) is a drawing illustrating the first to the third embodiments of the fluorescent immunostaining method according to the present invention.
Figs. 2(D) to 2(G) are drawings illustrating the fourth to the seventh embodiments of the fluorescent immunostaining method according to the present invention.
Figs. 3(H) to 3(K) are drawings illustrating the eighth to the eleventh embodiments of the fluorescent immunostaining method according to the present invention.
Figs. 4(L) to 4(O) are drawings illustrating the twelfth to the fifteenth embodiments of the fluorescent immunostaining method according to the present invention.

### Description of Embodiments

The fluorescent immunostaining method according to the present invention is a fluorescent immunostaining method including a first reaction step of specifically binding and fixing a primary antibody to an antigen as a staining subject, a second reaction step of specifically binding a secondary antibody to the bound and fixed primary antibody, and a third reaction step of fluorescence labeling the secondary antibody with a fluorescent nanoparticle, characterized in that plural secondary antibodies are fixed to one molecule of the antigen by way of the primary antibodies.

Hereinbelow, each element used for the fluorescent immunostaining method according to the present invention is explained first.

### [Antigen]

The antigen is a biological substance, in particular, a protein (antigen), expressed in sample slide, and it indicates a subject for immunostaining using fluorescent label to have quantification or detection mainly from the viewpoint of pathological diagnosis. Examples of the antigen to which the present invention can be applied include a biological substance which is expressed in the cell membranes of various cancer tissues and can be utilized as biomarkers, such as PD-L1 (Programmed cell death 1 ligand 1) of transmembrane protein which shows increased expression in cancer cells and are related to immune checkpoint, or growth factor receptors such as EGFR (HER1) (Epidermal Growth Factor Receptor), HER2 (Human Epidermal Growth Factor Receptor), HER3, HER4, VEGFR (Vasular Endothelial Growth Factor Receptor), IGFR (Insulin-like Growth Factor Receptor), or HGFR (Hepatocyte Growth Factor Receptor)), and proteins serving as immune system receptors such as PD-1 (Programmed cell death 1) which is a receptor of PD-L1 as an important inhibitory immune checkpoint molecule present on a surface of T cells. Examples of EGFR/HER include EGFR/HER1 (also called "ErbB1") which is overexpressed in cancer tissues such as colon cancer, EGFR2/HER2 (also called "ErbB2" or "neu") which is overexpressed in cancer tissues such as breast cancer, EGFR3/HER3, and EGFR4/HER4. Examples of VEGFR include VEGFR-1 (also called "Flt-1") and VEGFR-2 (also called "Flt-2" or "KDR"), which show enhanced expression in vascular endothelial cells of cancer tissues such as liver cancer and esophageal cancer, and VEGFR-3 (also called "Flt-4") which shows enhanced expression in lymphatic endothelial cells. For example, PD-L1 is suitable as an antigen when the method of the present invention is performed in pathological diagnosis relating to cancer.

### [Primary antibody]

The primary antibody is an antibody which recognizes a unique epitope in antigen, and it may be a free primary antibody or a primary antibody either directly or indirectly fixed on a surface of a nanoparticle not containing a fluorophore that is described below (non-fluorescent nanoparticle). When a polyclonal antibody is used as primary antibody, a common polyclonal antibody that is produced by having a specific sequence region containing the epitope of an antigen molecule as an immune antigen can be used, but it is not limited thereto. For example, a mixture of two or more kinds of a monoclonal antibody can be used as a substitute (equivalent) of a polyclonal antibody. In that case, it is preferable to have a combination of monoclonal antibodies in which each antibody specifically binds to a different epitope.

As long as the secondary antibody can bind to the primary antibody already bound to an antigen, the primary antibody is not required to be those with full length like natural antibody, and it may be either a fragment or a derivative of an antibody. Namely, the term "antibody" used herein encompasses not only the full-length antibody but also an antibody fragment and a derivative like a chimeric antibody (humanized antibody or the like) and a polyfunctional antibody.

Furthermore, the kind of the animal (immune animal) for producing the primary antibody is not particularly limited, and the animal may be selected from mice, rats, guinea pigs, rabbits, goats, sheep and the like as in conventional cases.

As for the primary antibody, IgG antibody can be preferably used. For example, in a case in which PD-L1 is an antigen, anti PD-L1 antibody can be used, and in a case in which HER2 is an antigen, anti HER2 antibody can be used.

### <Primary antibody directly bound to surface of non-fluorescent nanoparticle>

Other than a free antibody, the primary antibody can be a primary antibody that is directly fixed on a surface of a non-fluorescent nanoparticle (to be described later) with diameter of nanometer order (mainly, 1 to 1000 nm) as described above.

### (Non-fluorescent nanoparticle)

The non-fluorescent nanoparticle is a nanoparticle of nanometer order which does not contain a fluorescent substance, and it may be either an organic nanoparticle or an inorganic nanoparticle as long as a functional group capable of binding to the functional group of the primary antibody is included therein or can be introduced thereto.

As for the organic nanoparticle, a nanoparticle made of a resin like polystyrene, melamine resin, epoxy resin, acyl resin, and urea resin can be used, for example.

Basically, except that a fluorophore is not used (not added), the non-fluorescent nanoparticle can be produced in the same manner as the method for producing a fluorescent nanoparticle described below.

Average particle size of the non-fluorescent nanoparticle is one of the elements which determine the number of non-reacted primary antibody (= amount available for binding with secondary antibody), and also the detection sensitivity. Due to such reasons, it is preferable that the average particle size of the non-fluorescent nanoparticle is controlled within a suitable range during the production. The average particle size of the non-fluorescent nanoparticle is preferably 50 nm to 200 nm, although it is not particularly limited as long as the sensitivity for detecting a signal is not impaired.

In a case in which an organic non-fluorescent nanoparticle is to be produced, it is preferable to use emulsion polymerization in which a resin monomer is polymerized in emulsified micelles (W/O emulsion) among the polymerization methods that are generally known. That is because, according to emulsion polymerization, micelle size (that is, average particle size of non-fluorescent nanoparticle) can be suitably adjusted to a desired size according to modification of the type or amount of a surfactant to be added.

### (Binding between non-fluorescent nanoparticle and primary antibody)

Binding between the non-fluorescent nanoparticle and primary antibody can be carried out by using a known method by which proteins are bound to an organic substance or an inorganic substance. Mode of the binding is not particularly limited. For example, by utilizing a specific binding between biological molecules (e.g., hapten-anti hapten antibody or the like), the non-fluorescent nanoparticle and primary antibody can bind to each other, or binding based on covalent bond, ionic bond, hydrogen bond, coordination bond, physical adsorption, chemical adsorption, or the like can be employed. In case of a covalent bond, a covalent bond between SH group (antibody side)-maleimide group or the like (nanoparticle side) is preferable from the viewpoint of having easy binding.

Examples of a functional group which can replace the maleimide group include an aldehyde group and a bromoacetamide group (iodoacetamide group and bromoacetamide group) which can undergo a binding reaction with SH group. However, the maleimide group is more preferable than those functional groups since it has favorable reactivity with SH group and the reagent required for introduction is easily obtainable.

For example, as a method of producing a non-fluorescent nanoparticle using polystyrene as a mother compound and introducing a maleimide group to the non-fluorescent nanoparticle, there is a method of introducing a maleimide group based on Cl-exchange etherification between the OH group of N-hydroxymethylmaleimide and chloromethyl group while the phenyl group of polystyrene chain is already cholomethylated, as it is described in JP 2007-23120 A.

Meanwhile, in a case in which the non-fluorescent nanoparticle is an inorganic nanoparticle, a maleimide group or the like can be introduced by using a coupling agent like silane coupling agent.

### (Determination of introduced functional group)

Determination of the introduction of a functional group (group capable of binding to SH group or the like) to a surface of the non-fluorescent nanoparticle and also the quantification of the functional group can be carried out by a method of measuring peaks and an area at a wavelength corresponding to the binding based on FT-IR, or a kit for quantifying the functional group, for example.

When the introduced maleimide group (binding group) is quantified, the quantification can be made by using "Amplite™ Fluorimetric Maleimide Quantification Kit" (manufactured by Cosmo Bio Co., Ltd.).

When the introduced aldehyde group is quantified, the quantification method using 2,4-dinitrophenylhydrazine (DNPH) can be mentioned. It is a method in which the aldehyde group on a surface of the non-fluorescent nanoparticle is reacted with DNPH to form a DNPH derivative, which is then subjected to high performance liquid chromatography (HPLC) to have adsorption on column followed by elution, and the aldehyde group is analyzed and quantified from the absorption amount of the absorbance (Abs. 360 nm) of the elution fraction corresponding to the DNPH derivative.

In case of the introduced haloacetamide group (iodoacetamide group and bromoacetamide group), the introduction amount can be examined, according to a known method, by quantifying the halogen which is actually generated in accordance with the reaction with a SH group.

### (Treatment for introducing SH group to primary antibody)

When a disulfide bond (-S-S-) present on an antibody part which does not contribute to the antigen-antibody reaction is converted to a thiol group (-SH) after reducing the primary antibody, it is possible to have a reaction with a functional group (e.g., thiol group) introduced to a surface of a non-fluorescent nanoparticle so that the antibody can be directly bound to the non-fluorescent nanoparticle. Herein, it is necessary to carry out the reduction so as not to impair the binding reactivity of Fab region in at least one direction of the primary antibody. For example, it is described in JP 2003-509680 A that, by using 2-aminoethanethiol among disulfides for connecting heavy chains of a primary antibody, only the disulfide bond in Fc region can be selectively reduced.

Examples of a suitable reducing agent include 2-aminoethanethiol and 2-mercaptoethanol. It is preferable to use a pH buffer (e.g., PBS) such that the reduction is carried out in neutral-range pH buffer, in which the reducing agent to be used can exhibit the reducing powder.

Since the reduction amount of a disulfide bond (S-S bond) included in an antibody molecule increases as the pH during reduction (reducing pH) shifts to an alkali side, from the viewpoint of not impairing the reactivity of a primary antibody, the reducing pH is preferably adjusted, within a pH range in which the reducing ability of a reducing agent is exhibited, such that introduction is made to have the smallest amount of SH group possible in one antibody molecule. As for the reducing pH, when 2-aminoethanethiol is used, for example, by adjusting it to pH of from 6.0 to 7.5, suitable reducing power can be obtained. When 2-mercaptoethanol is used, it is pH of from pH 7.0 to 8.5. Conditions for the reducing reaction include 8 to 36 hours at 4°C to 8°C. Furthermore, it is preferable that the molar concentration of the reducing agent is 100,000,000,000 to 10,000,000,000,000 mol relative to 1 mol of the antibody. Quantification of the SH group in antibody molecule can be carried out by a known method like a method of using a kit for SH group quantification including a known agent for SH group quantification (e.g., 5,5'-dithiobis(2-nitrobenzoic acid)), Dojindo product code: D029, Product name: DTNB), for example.

### (Binding treatment)

Binding between the antibody and non-fluorescent nanoparticle is preferably achieved by a binding reaction for 1 to 12 hours at room temperature (1 to 40°C) while the non-fluorescent nanoparticle : antibody (molar ratio) is set within a range of from 1 : 100,000 to 1 : 100,000,000. In this case, the binding reaction is preferably carried out in a pH buffer (e.g., PBS buffer) containing a chelating agent (e.g., EDTA).

### (Another method for binding)

Furthermore, although the above example relates to a method of binding between a functional group like maleimide group and a SH group of an antibody, as a substitute, a method of reacting a non-fluorescent nanoparticle having an amino group with sulfo-SMCC followed by additional binding reaction with a SH group in the antibody to bind the primary antibody to non-fluorescent nanoparticle can be also used.

### (Still another method for binding)

As described below, it is also possible that an amino group is introduced to a non-fluorescent nanoparticle, and after further carrying out a treatment of converting the amino group to a maleimide group, binding to the primary antibody group having SH group is achieved.

### (Introduction of amino group to non-fluorescent nanoparticle)

When the non-fluorescent nanoparticle is made of a melamine resin, for example, it is preferable to carry out a treatment for introducing an amino group to the non-fluorescent nanoparticle. That is because, although the melamine resin has an amino group by itself, as a result of carrying out a treatment for introducing an amino group, an amino group is additionally added to a surface of the fluorescent nanoparticle made of melamine resin, and thus the reactivity with a SH group or the like which is introduced to an antibody can be increased as described below.

As for the treatment for introducing an amino group, there is an example in which the fluorescent nanoparticle is dispersed in a predetermined solvent (e.g., water), and the resulting dispersion is added with a reagent for introducing an amino group and heated and stirred for 10 to 30 minutes at 60 to 80°C.

As for the reagent for introducing an amino group described above, 1,2-bis(2-aminoethoxy)ethane (BAEE), tris(2-aminoethyl)amine (TAEA), or the like can be used.

Introduction of an amino group can be confirmed by FT-IR or XPS measurement.

### (Conversion of amino group to maleimide group or the like)

When a linker which has a functional group capable of reacting with an amino group (NHS group or the like) at one end and also a functional group capable of reacting with a SH group of the primary antibody (maleimide group or the like) at the other end is reacted with an amino group of the non-fluorescent nanoparticle, a maleimide group or the like can be introduced to the non-fluorescent nanoparticle. Examples of the linker which may be used include "SM(PEG)n" (n = 2, 4, 6, 8, 12, or 24) (Thermo Fisher Scientific Inc.).

### (Binding between non-fluorescent nanoparticle and primary antibody)

By adjusting the mass ratio of a non-fluorescent nanoparticle to an antibody having fluorescent SH group to 1 : 500 to 1 : 1500, mixing them in PBS, and carrying out a reaction to bind both molecules for 1 hour at room temperature, the non-fluorescent nanoparticle and antibody can bind to each other.

Meanwhile, when the primary antibody is to be bound to an inorganic non-fluorescent nanoparticle, the binding may be achieved by using a coupling reagent. Herein, examples of the coupling agent which may be used include a silane-based, a titanate-based, an aluminum-based, and a zirconium-based coupling agent, and two or more kinds of a coupling agent may be used in combination. With regard to the order of the reaction between the non-fluorescent nanoparticle and a silane coupling agent, a known method can be used. In addition, a condensation agent like EDC may be used, if necessary.

### (Binding density)

Binding density of the primary antibody on a surface of the non-fluorescent nanoparticle is not particularly limited as long as the binding reactivity between the primary antibody on a surface of the non-fluorescent nanoparticle and the antigen or secondary antibody is maintained. However, it is preferably from 2 × 10⁻¹⁴ mol/l mm² (particle surface) to 5 × 10⁻⁷ mol/l mm² (particle surface) (in case of polyclonal antibody, it is a value in total). Since the possible binding amount of the secondary antibody on a surface of the non-fluorescent nanoparticle changes in accordance with the binding density of the primary antibody and the detection sensitivity changes accordingly, the detection sensitivity may be controlled (enhanced or reduced) by adjusting the binding density within a range in which the binding reactivity is maintained. For example, when the antigen cannot be detected due to low sensitivity, the detection sensitivity can be enhanced by carrying out an adjustment for increasing the binding density like increasing the number of a functional group to be introduced (SH group or the like) and vice versa.

Furthermore, the binding density is calculated as follows. Amount of the primary antibody on a surface of the non-fluorescent nanoparticle after a purification treatment is measured by BCA method or the like, and the amount is divided by weight molecular weight of the primary antibody to calculate mole number. Meanwhile, when the half value of the average particle size of non-fluorescent nanoparticle, which is measured by electron microscopy like SEM, is taken as radius (r), the surface area (mean) of one non-fluorescent nanoparticle is calculated from the equation for calculating surface area of sphere (4πr²) and then multiplied by number of the particles in a dispersion of a subject measured by using particle counter or the like to calculate a total surface area of a non-fluorescent nanoparticle, and the calculated value is divided by the mole number calculated in the above, the binding density of the primary antibody on a surface of the non-fluorescent nanoparticle can be calculated.

### [Secondary antibody]

The secondary antibody means an antibody which specifically recognizes an antigen non-reacting part of the antigen-fixed primary antibody (Fc, F(ab), or F(ab')) and binds to a part or whole of the primary antibody, and it is an antibody that does not bind to the antigen, and is already bound to the fluorescent nanoparticle or to be subsequently bound thereto.

In an embodiment in which the primary antibody is used in a state that it is bound to a surface of the non-fluorescent nanoparticle, the secondary antibody will react with a non-reacted part (Fc, F(ab), or F(ab')) of a primary antibody present on a surface of the non-fluorescent nanoparticle fixed to the antigen (primary antibody not bound to the antigen).

As for the secondary antibody, IgG antibody or the like can be used. In general, the secondary antibody is produced from an immune animal which is different from the animal of the primary antibody, and it is produced such that it can recognize the antibody (Fc region or the like) of animal species of the primary antibody. The kind of the animal (immune animal) used for producing the primary and secondary antibodies is not particularly limited, and the selection is preferably made from mice, rats, guinea pigs, rabbits, goats, sheep and the like as in conventional cases.

### (Binding between secondary antibody and fluorescent nanoparticle)

Binding between the secondary antibody and fluorescent nanoparticle can be direct binding between functional groups as it has been described regarding the binding between the primary antibody and non-fluorescent nanoparticle. Alternatively, it can be an indirect linking using a linker and a first and a second binding group material as described below. In a case in which the secondary antibody is bound to a surface of the fluorescent nanoparticle, the binding density of the secondary antibody to a surface of the fluorescent nanoparticle is not limited as long as the binding reactivity of the primary antibody fixed to an antigen is maintained. However, it is preferably from 2 × 10⁻¹⁴mol/l mm² (particle surface) to 5 × 10⁻⁷ mol/1 mm² (particle surface) (same binding density range as the primary antibody). Furthermore, calculation of the antibody binding density of the secondary antibody is the same as those described above in relation to the non-fluorescent nanoparticle. In particular, when plural primary antibodies are bound to a surface of the non-fluorescent nanoparticle, the binding density of the secondary antibody is preferably 1.0 to 1.5 times the binding density of the primary antibody.

### (Indirect binding between secondary antibody and fluorescent nanoparticle)

As for the method for having indirect binding between the secondary antibody and fluorescent nanoparticle, it may be a method in which a linker is bonded to each of the secondary antibody and fluorescent nanoparticle and the secondary antibody is bound to the fluorescent nanoparticle based on specific binding reaction between the first and second binding group materials that are provided at tip part of the linker, respectively. Examples of the first and second binding group materials include a combination of biomolecules like biotin : avidin. It is also possible to have a combination of a known substance other than biotin : avidin (e.g., hapten-anti hapten antibody). Furthermore, the first and second binding group materials do not include those reacting with the primary or secondary antibody, or the antigen.

When the aforementioned secondary antibody is modified with the first binding group material by way of a linker, it is preferable to use a known linker which has a functional group available for the binding and a first binding group material at both ends. Such linker is commercially available, and it can be purchased from Thermo Fisher Scientific Inc., for example.

For example, by using a linker which has a maleimide group at one end and biotin at the other end and binding the maleimide group at one end to a SH group of an antibody which is formed by reduction as described above, biotin can be bonded to the secondary antibody by way of PGE. Examples of the linker include "Biotin-PEG6-NH-Mal" (manufactured by PurePEG LLC) and EZ-Link Maleimide-PEG2-Biotin (manufactured by Thermo Fisher Scientific Inc.).

Meanwhile, with regard to the binding for modifying the fluorescent nanoparticle with the second binding group material by way of a linker, for example, SM(PEG)₆ is a linker which has a NHS group at one end and a maleimide group at the other end, and therefore, after binding the NHS group at one end to an amino group introduced to the fluorescent nanoparticle, the maleimide group at the other end of a linker is bonded to a SH group of streptavidin (SA) which is formed by reduction described above, the second binding group material (in this case, SA) can be bonded to a fluorescent nanoparticle.

Examples of the linker include "SM(PEG)n" (n = 2, 4, 6, 8, 12, or 24) (Thermo Fisher Scientific Inc.). Furthermore, as for the reagent for introducing an amino group described above, 1,2-bis(2-aminoethoxy)ethane (BAEE), tris(2-aminoethyl)amine (TAEA), or the like can be used.

Finally, according to the binding between the first binding group material and the second binding group material, the fluorescent nanoparticle is bonded to the secondary antibody to yield a fluorescence labeled secondary antibody. Furthermore, with regard to the binding between the first binding group material and the second binding group material, the secondary antibody can be fluorescence labeled at desired time point either before and after the second reaction step in which binding of the secondary antibody to the primary antibody is carried out. Furthermore, the binding density of the secondary antibody relative to the fluorescent nanoparticle and the use ratio of the antibody are the same as those described for the non-fluorescent nanoparticle or primary antibody.

### (Direct binding between secondary antibody and fluorescent nanoparticle)

Unlike the above, it is also possible to have a method in which the secondary antibody and fluorescent nanoparticle are connected to each other by having one linker interposed therebetween without having a binding between the first and second binding group materials. In that case, the third reaction step is carried out before the first reaction step (before starting the immunostaining step), and the secondary antibody is bound to the fluorescent nanoparticle to be in a fluorescence labeled state.

For example, when a linker which has a maleimide group at one end and a NHS group at the other end is used to have a binding between the NHS group and the amino group of the aforementioned fluorescent nanoparticle, and the malemide group is bonded to a SH group of the secondary antibody that is formed by reduction as described above, the fluorescent nanoparticle and secondary antibody can be bound to each other. Examples of the linker which may be used include "SM(PEG)n" (n = 2, 4, 6, 8, 12, or 24) (Thermo Fisher Scientific Inc.).

Furthermore, the binding density of the secondary antibody relative to the fluorescent nanoparticle and the use ratio in this case are the same as those described for the direct binding of the primary antibody to a fluorescent nanoparticle.

### (Length and material of linker)

With regard to a complex of the secondary antibody and fluorescent nanoparticle, which are bound to each other by way of a linker, length of the linking part originating from the linker is preferably 15 angstrom or more and 1000 angstrom or less, and particularly preferably 30 angstrom or more and 65 angstrom or less. Examples of the linker which may be used include a hydrophilic polymer like polyethylene glycol, polypropylene glycol, ficoll, and polyvinyl alcohol, although it is not particularly limited thereto.

### [Method for producing non-fluorescent nanoparticle]

As described above, the non-fluorescent nanoparticle can be produced in the same manner as above except that a fluorescent dye is used for the fluorescent nanoparticle.

Specifically, as a production method for producing an organic nanoparticle as the non-fluorescent nanoparticle, for example, an emulsifying agent for emulsion polymerization is first added to an aqueous dispersion medium like distilled water, and after raising the temperature to a predetermined temperature (e.g., 60 to 80°C) under heating and stirring, a resin material is added to the solution.

Furthermore, by carrying out heating and stirring after adding an aqueous solution of a surfactant to the solution (e.g., 50 minutes at 70°C → 20 minutes at 90°C), the non-fluorescent nanoparticles are granulated by emulsion polymerization.

By carrying out a washing treatment to remove impurities like excess resin materials and fluorescent dyes from a dispersion of the obtained melamine-based fluorescent nanoparticles, non-fluorescent nanoparticles can be produced.

As for the emulsifying agent for emulsion polymerization, a known emulsifying agent can be used, and examples thereof include an anionic, a non-ionic, and a cationic emulsifier. When a (cationic) thermosetting resin having a positively charged substituent or region is synthesized, it is preferable to use an anionic or a non-ionic surfactant. On the other hand, when an (anionic) thermosetting resin having a negatively charged substituent or region is synthesized, it is preferable to use a cationic or a non-ionic surfactant.

Examples of the anionic surfactant include sodium dodecybenzenesulfonate (product name: NEOPELEX, manufactured by Kao Corporation). Examples of the non-ionic surfactant include polyoxyethylene alkyl ether-based compound (product name: EMULGEN, manufactured by Kao Corporation), polyvinyl pyrrolidone (PVP), and polyvinyl alcohol (PVA). Examples of the cationic surfactant include dodecyl trimethylammonium bromide.

Furthermore, as for the resin material, a known resin monomer can be used, and examples thereof include a melamine resin material "NIKALAC MX-035" (manufactured by NIPPON CARBIDE INDUSTRIES CO., INC.).

Furthermore, as for the surfactant, a known surfactant can be used, and examples thereof include dodecybenzenesulfonate (manufactured by KANTO CHEMICAL CO., INC.).

Particle diameter of the non-fluorescent nanoparticle and variation coefficient thereof can be obtained by performing an observation by scanning electron microscopy or the like and calculating the average particle size and variation coefficient.

### [Fluorescent nanoparticle]

The fluorescent nanoparticle used in the present invention is preferably a "fluorescent substance-integrated nanoparticle" that is capable of emitting fluorescence with an intensity sufficient for exhibiting each antigen molecule as a bright spot.

The term "fluorescent substance" used herein refers to a substance whose electrons are excited when the substance is irradiated with an electromagnetic wave of a prescribed wavelength (X-ray, UV radiation or visible light) and absorbs the energy thereof and which releases an excess energy in the form of an electromagnetic wave during the transition from an excited state to the ground state, namely a substance which emits "fluorescence", and the substance can be directly or indirectly bound with the primary antibody. Furthermore, the term "fluorescence" has a broad meaning and encompasses phosphorescence which has a long emission lifetime sustaining the emission even after the irradiation with an electromagnetic wave for excitation is terminated; and also fluorescence in a narrow sense, which has a short emission lifetime.

### <Fluorescent substance-integrated nanoparticle>

The fluorescent substance-integrated nanoparticle which can be used in the present invention is a nano-sized particle having a structure in which a particle made of an organic or inorganic substance is a matrix and plural fluorescent substances (e.g., fluorescent dyes) are contained in the matrix and/or adsorbed on the surface of the matrix. In this case, it is preferred that the matrix (e.g., a resin) and the fluorescent substances each include a substituent or moiety having an opposite electric charge and that an electrostatic interaction occurs therebetween.

Among matrices that can constitute the fluorescent substance-integrated nanoparticle, examples of an organic substance include resins that are generally classified into thermosetting resins, such as melamine resins, urea resins, aniline resins, guanamine resin, phenolic resins, xylene resins and furan resins; resins that are generally classified into thermoplastic resins, such as styrene resins, acrylic resins, acrylonitrile resins, AS resins (acrylonitrile-styrene copolymers) and ASA resins (acrylonitrile-styrene-methyl acrylate copolymers); other resins such as polylactic acids; and polysaccharides, and examples of an inorganic substance include silica and glass.

### • Semiconductor-integrated nanoparticle

Semiconductor-integrated nanoparticle has a structure in which semiconductor nanoparticle as fluorophore is contained in the above-described matrix and/or adsorbed on the surface of the matrix. The material constituting the semiconductor nanoparticle is not particularly limited and may be a material containing a Group II-VI compound, a Group III-V compound or a Group IV element, examples of which include CdSe, CdS, CdTe, ZnSe, ZnS, ZnTe, InP, InN, InAs, InGaP, GaP, GaAs, Si, and Ge. Furthermore, when the semiconductor is contained in the matrix, the semiconductor is preferably dispersed in the matrix, and it may or may not be chemically bound with the matrix itself.

### • Fluorescent dye-integrated nanoparticle

Fluorescent dye-integrated nanoparticle has a structure in which a fluorescent dye is contained in the above-described matrix and/or adsorbed on the surface of the matrix. The fluorescent dye is not particularly limited, and examples thereof include rhodamine-based dye molecules, squarylium-based dye molecules, cyanine-based dye molecules, aromatic ring-containing dye molecules, oxazine-based dye molecules, carbopyronine-based dye molecules, and pyrromethene-based dye molecules. Alternatively, for example, Alexa Fluor (registered trademark, manufactured by Invitrogen Corp.)-based dye molecules, BODIPY (registered trademark, manufactured by Invitrogen Corp.)-based dye molecules, Cy (registered trademark, manufactured by GE Healthcare)-based dye molecules, DY (registered trademark, manufactured by Dyomics GmbH)-based dye molecules, HiLyte (registered trademark, manufactured by AnaSpec Inc.)-based dye molecules, DyLight (registered trademark, manufactured by Thermo Fisher Scientific Inc.)-based dye molecules, ATTO (registered trademark, manufactured by ATTO-TEC GmbH)-based dye molecules, and MFP (registered trademark, manufactured by Mobitec Co., Ltd.)-based dye molecules can be used as well. The generic names of these dye molecules are assigned based on the main structure (skeleton) or registered trademark of the respective compounds; therefore, those of ordinary skill in the art should be able to properly understand the scope of fluorescent dyes belonging to the respective generic names without having to bear undue trial and error. Furthermore, when the fluorescent dye is contained in the matrix, the fluorescent dye is preferably dispersed in the matrix, and it may or may not be chemically bound with the matrix itself.

Fluorescent substance-integrated nanoparticle can be produced in accordance with a known method (see, for example, JP 2013-57937 A). More specifically, for example, fluorescent substance-containing silica particles in which silica is used as a matrix and fluorescent substances are contained therein can be produced by adding dropwise a solution, in which inorganic semiconductor nanoparticle, a fluorescent substance such as an organic fluorescent dye and a silica precursor such as tetraethoxysilane are dissolved, to a solution in which ethanol and ammonia are dissolved, and subsequently hydrolyzing the silica precursor.

Meanwhile, fluorescent substance-containing resin particles in which a resin is used as a matrix and a fluorescent substance is adsorbed on the surface of the resin particles or contained in the resin particles can be produced by preparing in advance a solution of the resin or a dispersion of fine particles of the resin, adding thereto inorganic semiconductor nanoparticle and a fluorescent substance such as an organic fluorescent dye, and subsequently stirring the resultant. Alternatively, fluorescent substance-containing resin particles can also be produced by adding a fluorescent dye to a solution of a resin material and then allowing polymerization reaction to proceed. For example, in cases where a thermosetting resin such as a melamine resin is used as a matrix resin, organic fluorescent dye-containing resin particles can be produced by heating a reaction mixture, which contains a raw material of the resin (a monomer, an oligomer or a prepolymer, such as methylolmelamine obtained by condensation of melamine and formaldehyde), an organic fluorescent dye, and preferably further a surfactant and a polymerization reaction accelerator (e.g., an acid), and thereby allowing polymerization reaction to proceed by an emulsion polymerization method.

Furthermore, in cases where a thermoplastic resin such as a styrene-based copolymer is used as a matrix resin, organic fluorescent dye-containing resin particles can be produced by heating a reaction mixture, which contains a raw material of the resin, an organic fluorescent dye (as a resin material monomer, a monomer bound with an organic fluorescent dye through a covalent bond or the like in advance may also be used) and a polymerization initiator (e.g., benzoyl peroxide or azobis-isobutyronitrile), and thereby allowing polymerization reaction to proceed by a radical polymerization method or an ionic polymerization method.

Examples of the fluorescent substance to be contained in a matrix include, in addition to the above-described semiconductor nanoparticles and fluorescent dyes, "long-afterglow fluorophors" that include Y₂O₃, Zn₂SiO₄ or the like as a matrix and Mn²⁺, Eu³⁺ or the like as an activator.

The average particle size of the fluorescent substance-integrated nanoparticle (particularly, fluorescent dye-containing resin particle obtained by a production method as described above) is not particularly limited as long as it is suitable for immunostaining of a sample slide; however, taking into consideration, for example, the easiness of detection as bright spots, the average particle size is usually 10 to 500 nm, and preferably 50 to 200 nm. Furthermore, the variation coefficient, which represents the variation in the particle size, is usually 20% or less, and preferably 5 to 15%. Fluorescent substance-integrated nanoparticle satisfying these conditions can be produced by adjusting the production conditions. For example, in the production of such fluorescent substance-integrated nanoparticle by an emulsion polymerization method, the particle size can be adjusted by changing the amount of a surfactant to be added and, in general, when the amount of the surfactant is relatively large with respect to the amount of the parent materials of the fluorescent substance-integrated nanoparticle, the particle size tends to be small, whereas when the amount of the surfactant is relatively small, the particle size tends to be large. In addition, as for the surfactant, those described in relation to the production of the non-fluorescent nanoparticle can be also used.

Furthermore, the size of a fluorescent substance-integrated nanoparticle can be determined by taking an electron micrograph thereof using a scanning electron microscope (SEM), measuring the cross-sectional area of the fluorescent substance-integrated nanoparticle and then calculating the particle size as the diameter of a circle corresponding to the thus-measured cross-sectional area with an assumption that the cross-sectional shape is a circle. With regard to the average particle size and variation coefficient of a group consisting of plural fluorescent substance-integrated nanoparticles, after determining the particle size for a sufficient number (e.g., 1,000) of the fluorescent substance-integrated nanoparticles in the above-described manner, the average particle size is calculated as the arithmetic mean of the thus-obtained values, and the variation coefficient is calculated using the following equation: 100 × (standard deviation of particle size)/(average particle size).

### [Fluorescent immunostaining method]

The fluorescent immunostaining method according to the present invention is a fluorescent immunostaining method including a first reaction step of specifically binding and fixing a primary antibody to an antigen as a staining subject, a second reaction step of specifically binding a secondary antibody to the bound and fixed primary antibody, and a third reaction step of fluorescence labeling the secondary antibody with a fluorescent nanoparticle, characterized in that plural secondary antibodies are fixed to one molecule of the antigen by way of the primary antibodies.

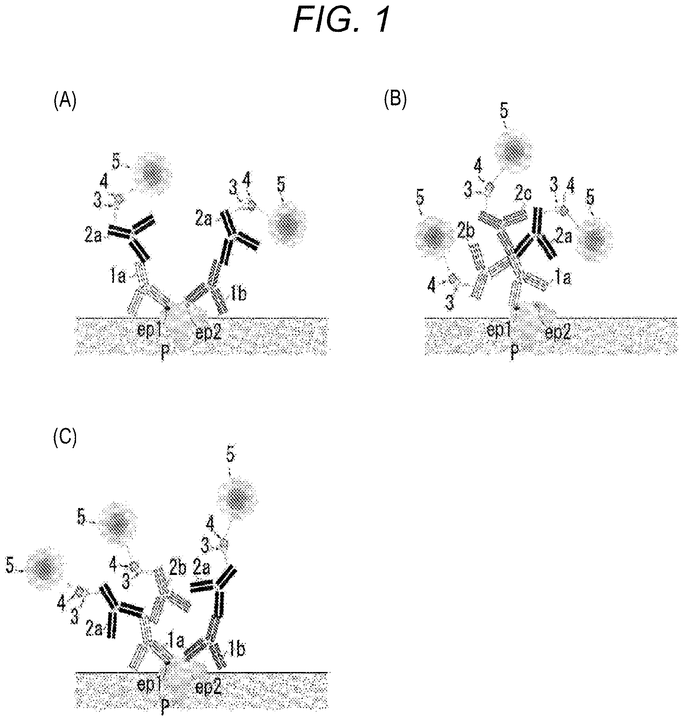

As an embodiment for fixing plural antibodies to one molecule of the antigen, there are embodiments (A) to (O) as the first to the fifteenth embodiments that are described in the following [Table 1] when the primary antibody and secondary antibody are used (see, (A) to (O) of Figs. 1 to 4 for each).

**[Table 1]**

| | | Secondary antibody | | | | |
|---|---|---|---|---|---|---|
| | | | Free | | Surface of fluorescent nanoparticle | |
| | | | Monoclonal antibody | Polyclonal antibody | Monoclonal antibody | Polyclonal antibody |
| Primary antibody | Free ^{(*)} | Monoclonal antibody | - Comparative Example 1 | Second embodiment Fig. 1(B) Example 1 | Fourth embodiment Fig. 2(D) | Fifth embodiment Fig. 2(E) |
| | | Polyclonal antibody | First embodiment Fig. 1(A) Example 2 | Third embodiment Fig. 1(C) Example 3 | Sixth embodiment Fig. 2(F) | Seventh embodiment Fig. 2(G) |
| | Surface of non-fluorescent nanoparticle *1 | Monoclonal antibody | Eighth embodiment Fig. 3(H) Example 4 | Ninth embodiment Fig. 3(I) Example 5 | Twelfth embodiment Fig. 4(L) | Thirteenth embodiment Fig. 4(M) |
| | | Polyclonal antibody | Tenth embodiment Fig. 3(J) Example 6 | Eleventh embodiment Fig. 3(K) Example 7 | Fourteenth embodiment Fig. 4(N) | Fifteenth embodiment Fig. 4(O) |

| | | | | | | |
|---|---|---|---|---|---|---|
| (*) "free" means a free state with no binding on a surface of a non-fluorescent nanoparticle. | | | | | | |

### (Sample slide)

It is possible that, according to a known method, cells that are either positive or negative in terms of the antigen (PD-L1 or the like) are acclimated by subculture, fixed with formalin (24 to 48 hours), embedded in paraffin, and cut with a microtome to give a "sample slide". The "sample slide" can be also prepared by a known method for producing a tissue specimen. The "sample slide" prepared from any one of tissue specimen and cultured cells are an application subject of the present invention.

Each embodiment of the fluorescent immunostaining method of the present invention is explained hereinbelow while taking an example in which staining of a sample slide is carried out.

### [First embodiment]

The fluorescent immunostaining method of the first embodiment includes at least immunostaining steps in which, with regard to the sample slide, a first reaction step of binding a polyclonal antibody as a free primary antibody to an antigen, a second reaction step of binding a free monoclonal antibody as a secondary antibody to the primary antibody, and a third reaction step of fluorescence labeling the secondary antibody are carried out (see, Fig. 1(A)).

Hereinbelow, explanations are given for an example which includes a deparaffinization treatment step and an activation treatment step for a sample slide, the third reaction step for labeling the secondary antibody with a fluorescent nanoparticle, and evaluation (evaluation step or the like) of the sample slide after carrying out an optional step (treatment step for morphological observation or the like). Furthermore, the third reaction step may be either before or after the first reaction step or the second reaction step.

### (1. Sample preparation step)

### (1-1. Deparaffinization treatment)

The sample slide is immersed in xylene contained in a vessel to remove paraffin. The temperature for this process is not particularly limited and may be room temperature. The immersion time is preferably 3 minutes or longer but not longer than 30 minutes. If necessary, xylene may be replaced with fresh xylene during the immersion.

Then, the sample slide is immersed in ethanol contained in a vessel to remove xylene. The temperature of this process is not particularly limited and may be room temperature. The immersion time is preferably 3 minutes or longer but not longer than 30 minutes. If necessary, ethanol may be replaced with fresh ethanol during the immersion.

The sample slide is immersed in water contained in a vessel to remove ethanol. The temperature of this process is not particularly limited and may be room temperature. The immersion time is preferably 3 minutes or longer but not longer than 30 minutes. If necessary, water may be replaced with fresh water during the immersion.

### (1-2. Activation treatment)

In accordance with a known method, a biological substance of interest is subjected to an activation treatment. The activation conditions are not particularly defined here; however, as an activation liquid, for example, 0.01 M citrate buffer (pH 6.0), 1 mM EDTA solution (pH 8.0), 5% urea or 0.1 M Tris-HCl buffer can be used. As a heating device, for example, an autoclave, a microwave oven, a pressure cooker, or a water bath can be used. The temperature is not particularly limited, and the activation may be performed at room temperature. The heating can be performed at a temperature of 50 to 130°C for a time period of 5 to 30 minutes.

Subsequently, the sample slide after an activation treatment is immersed and washed in PBS contained in a vessel. The temperature of this process is not particularly limited and may be room temperature. The immersion time is preferably 3 minutes or longer but not longer than 30 minutes. If necessary, PBS may be replaced with fresh PBS during the immersion.

### (2. Immunostaining step)

### <First reaction step>

As illustrated in Fig. 1(A), the primary antibodies 1a and 1b as a free polyclonal antibody are bound to epitopes ep1 and ep2 of the antigen P in sample slide.

As for the reaction condition for the binding, the same condition as the reaction temperature, reaction time, and antibody concentration for an antibody reaction of common Western blotting can be employed. For example, after a solution of primary antibody (antibody concentration: 1 µg/mL) is applied on a sample slide, the reaction can be carried out by incubation overnight at 4°C. Furthermore, it is preferable to contain, in the solution of primary antibody, a blocking agent, pH buffer (TBS or the like), and surfactant (Tween20 or the like) in the same manner as the primary reaction of Western blotting. When the primary antibody is a polyclonal antibody like the present embodiment, it is preferable that a commercially available antibody is diluted at 1 : 100 to 1 : 1,000, and used at antibody concentration of 0.2 to 2 µg/mL.

Furthermore, number of the type of the primary antibody to be bound to an antigen may vary depending on the antigen epitope number or number of the type of the primary antibody prepared according to the antigen epitope number, and thus it is not limited to 2.

### <Second reaction step>

As illustrated in Fig. 1(A), Fc region (part or whole) of the primary antibodies 1a and 1b, or, although not illustrated, Fab region (part or whole) thereof not bound to the antigen P, that is, primary antibody part not contributing to the reaction with the antigen P, is bound with plural secondary antibodies 2a as a monoclonal antibody. Accordingly, 2 or more of the secondary antibody 2a are fixed to one antigen P.

Concentration of the secondary antibody 2a to be used is not particularly limited, as long as it allows the fixation. However, it is generally 0.8 to 1.2 µg/mL, and, by carrying a series of immunostaining steps with modification of dilution ratio, optimum concentration can be set. Furthermore, number of the type of the primary antibody to be bound to the antigen P is not limited to 2.

### <Third reaction step>

The secondary antibody 2a is bound with the fluorescent nanoparticle 5 to have fluorescence labeling. According to the example shown in Fig. 1(A), the fluorescence labeling is carried out based on the binding between the first and second binding group materials (e.g., biotin 3 and the streptavidin 4). In that case, it is preferable that the fluorescent nanoparticle 5 is used in the form of liquid in which tit is dispersed at 0.005 to 0.5 nM in physiological pH buffer like PBS. The dispersion liquid may suitably contain a blocking agent (BSA or the like) and a pH buffer (phosphoric acid). According to incubation for 1 to 5 hours at room temperature after applying the dispersion liquid on top of a sample slide, the fluorescent nanoparticle can be bound to the secondary antibody.

Furthermore, although the third reaction step may be carried out either before the first reaction step, or between the first reaction step and the second reaction step, it is preferably carried out after the second reaction step since the binding reactivity of the secondary antibody to the primary antibody is not likely to be impaired.

Furthermore, after each step of the first to the third reaction steps, it is preferable to have a washing step for washing the sample slide with PBS or the like. Prior to performing the above-described primary reaction treatment, it is preferred to add drops of a known blocking agent such as BSA-containing PBS or a surfactant such as Tween 20.

The conditions for each step of the immunostaining, such as the temperature and time of immersing the sample slide in each prescribed solution (reagent) for each of the first reaction step and the second reaction step, can be adjusted as appropriate in accordance with a conventional fluorescent immunostaining method such that appropriate signals can be obtained.

### (3. Treatment step after sampling)

After the completion of the immunostaining step, the sample slide is preferably subjected to treatments, such as fixation-dehydration, clearing, or sealing, such that the sample slide is made suitable for observation.

The fixation-dehydration treatment can be performed by immersing the sample slide in a fixation liquid (a cross-linking agent such as formalin, paraformaldehyde, glutaraldehyde, acetone, ethanol, or methanol). The clearing can be performed by immersing the thus-fixed and dehydrated sample slide in a clearing liquid (e.g., xylene). The sealing treatment can be performed by immersing the thus-cleared sample slide in a sealing medium. The conditions for performing these treatments, such as the temperature and time of immersing the sample slide in each prescribed treatment liquid, can be adjusted as appropriate in accordance with a conventional fluorescent immunostaining method such that appropriate signals can be obtained.

### (4. Optional step)

In the present invention, if necessary, a staining step for morphological observation can be incorporated so that the morphology of cells, tissues, organs and the like can be observed in a bright field. The staining step for morphological observation can be performed in accordance with a conventional method. For the morphological observation of a sample slide, eosin staining which stains cytoplasm, interstitial tissues, various fibers, erythrocytes and keratinocytes in red to dark red is typically employed. Furthermore, hematoxylin staining which stains cell nuclei, calcareous parts, cartilaginous tissues, bacteria and mucus in livid to light blue is also typically employed (a method of simultaneously performing these two staining processes is known as "hematoxylin-eosin staining" (HE staining)). In cases where the staining step for morphological observation is incorporated, it may be performed either after or before the immunostaining step.

### (5. Evaluation step)

### (5-1. Observation and image capturing)

In the observation and image capturing step, in the same visual field under a microscope at a desired magnification, the sample slide is irradiated with light having wavelength capable of exciting the fluorophores in the fluorescent nanoparticle, which are used in the immunostaining step, and each of the immunostained images produced by the emitted fluorescence is observed and captured. The excitation lights can be irradiated using, for example, a laser light source installed in a fluorescence microscope and, as required, an optical filter for excitation light which selectively transmits a prescribed wavelength. The immunostained images can be captured by using, for example, a digital camera mounted on a fluorescence microscope. In the process of capturing the immunostained images, by using, as required, an optical filter for fluorescence which selectively transmits a prescribed wavelength, immunostained images including only the desired fluorescence, from which undesired fluorescence, noise-causing exciting light and other lights are excluded, can be obtained.

### (5-2. Image processing and signal measurement)

In the image processing and measurement step, on the immunostained images captured for the antigen, the fluorescence labeled signals corresponding to the antigen are measured based on the results of image processing, and the fluorescence labeled signals corresponding to the antigen which is present in the cell membrane region are identified.

The fluorescence labeled signals are preferably handled in terms of the number of fluorescent bright spots.

Examples of software that can be used for the image processing include "ImageJ" (open source). The use of such an image processing software enables performing a process of extracting bright spots of a prescribed wavelength (color) from the immunostained images and determining the total brightness of the bright spots and a process of measuring the number of bright spots having a brightness of not less than a prescribed value, particularly those processes for carrying out the first and second embodiments that are described below, in a semi-automatic and prompt manner.

### (Evaluation regarding quantification property)

When the average number of bright spots (per cell) in sample slide which is positively stained by the fluorescent immunostaining method of this embodiment is examined, correlation of the expression amount with the fluorescent nanoparticle score (particle number identified under observation at the same conditions for each cell) is determined by using antigen-positive culture cells with known expression amount, and if the correlation of R² ≥ 0.70 is confirmed, it is possible to recognize that the quantification property is sufficient. Furthermore, because there is also a case in which one "bright spot" consists of plural "particles", if brightness of one "bright spot" is divided by mean brightness per "particle", "number of particles" included in the bright spot can be calculated.

### (Action and effect of first embodiment)

Hereinbelow, the action and effect of the first embodiment are explained in view of Fig. 1(A).

According to the fluorescent immunostaining method of the first embodiment, after each of the primary antibodies 1a and 1b as a polyclonal antibody specifically binds to each epitope of epitopes ep1 and ep2, the secondary antibodies 2a and 2b and the fluorescent nanoparticle are bound to the primary antibodies 1a and 1b in the order to allow a detection, and thus the detection noise can be suppressed at low level. Furthermore, as two fluorescent nanoparticles are fixed on each antigen, higher detection sensitivity can be obtained. Furthermore, number of the type of the primary antibody can be increased without being limited to 2. It is also possible that, depending on the number of an epitope of the antigen P and number of the type of the primary antibody to be used, number of the fluorescent nanoparticle 5 to be bound to one antigen P (that is, detection sensitivity) is controlled.

### [Second embodiment]

Because only the immunostaining step of the first embodiment is modified in the second embodiment, explanations for other steps are omitted, and only the immunostaining step is explained hereinbelow. Furthermore, other than those described below, the immunostaining step is the same as that of the first embodiment (ditto for other embodiments).

### <First reaction step>

As it is shown in Fig. 1(B), a solution of the primary antibody 1a as a free monoclonal antibody is applied on a sample slide, and, by carrying out an antigen and antibody reaction, the primary antibody 1a is allowed to bind only to epitope ep1 between plural epitopes ep1 and ep2 that are included in the antigen P of the sample slide.

### <Second reaction step>

As illustrated in Fig. 1(B), the second reaction step in which Fc region (part or whole) of the primary antibody 1a, or Fab region (part or whole) thereof not bound to the antigen P, that is, primary antibody part not contributing to the reaction with the antigen P, is bound with polyclonal antibodies (secondary antibody) 2a to 2c is carried out so that 2 or more of the secondary antibody are fixed to one antigen.

### <Third reaction step>

In the same manner as the first embodiment, each of the secondary antibodies 2a to 2c as a polyclonal antibody is bound with the fluorescent nanoparticle 5 by way of binding between the biotin 3 and the streptavidin 4 as it is shown in Fig. 1(B). As a result, the fluorescent nanoparticle 5 is bound and fixed to one antigen P by way of the secondary antibodies 2a to 2c.

### (Action and effect of second embodiment)

Hereinbelow, the action and effect of the second embodiment are explained.

According to the fluorescent immunostaining method of the second embodiment, because the primary antibody 1a is a monoclonal antibody, binding of the primary antibody 1a can be achieved in a state in which the specificity for the antigen P is maintained, in particular. In addition, since plural kinds of the fluorescent nanoparticle 5 and the secondary antibodies 2a to 2c as a polyclonal antibody are bound and fixed to the primary antibody 1a, high sensitization can be achieved while the specificity is preferably maintained.

Furthermore, because the binding between the fluorescent nanoparticle and secondary antibody is based on binding between the biotin 3 and the avidin 4, binding of the fluorescent nanoparticle to the secondary antibody can be achieved irrespective of the type of the fluorescent nanoparticle, and thus high sensitization can be suitably obtained. Furthermore, number of the type of the secondary antibody to be used is not limited to 3, and it may be either increased or decreased depending on the necessity of sensitization, and it may be 2 types, for example.

### [Third embodiment]

Because only the immunostaining step of the first embodiment is modified in the third embodiment, explanations for other steps are omitted, and only the immunostaining step is explained hereinbelow.

### <First reaction step>

As it is shown in Fig. 1(C), a solution of the primary antibodies 1a and 1b as a free polyclonal antibody is applied on a sample slide, and an antigen and antibody reaction is allowed to occur. Accordingly, each of the primary antibodies 1a and 1b binds to epitopes ep1 and ep2 that are included in the antigen P of the sample slide.

### <Second reaction step>

As illustrated in Fig. 1(C), the second reaction step in which Fc region (part or whole) of the primary antibodies 1a and 1b, or Fab region (part or whole) thereof not bound to the antigen P (that is, primary antibody part not contributing to the reaction with the antigen P) is bound with the secondary antibodies 2a and 2b as a free polyclonal antibody is carried out so that 2 or more of the secondary antibody are fixed to one antigen.

### <Third reaction step>

In the same manner as the first embodiment, each of the secondary antibodies 2a and 2b as a polyclonal antibody is bound with the fluorescent nanoparticle 5 by way of binding between the biotin 3 and the streptavidin 4, as it is shown in Fig. 1(C). As a result, 2 or more of the secondary antibodies 2a and 2b and fluorescent nanoparticle 5 are bound and fixed to one antigen P.

### (Action and effect of third embodiment)

Hereinbelow, the action and effect of the third embodiment are explained.

According to the fluorescent immunostaining method of the third embodiment, the primary antibodies 1a and 1b are bound to plural epitopes ep1 and ep2 included in the antigen P, and also, after binding the secondary antibodies 2a and 2b to multiple sites of the primary antibodies, the fluorescent nanoparticle 5 binds to each of the secondary antibodies 2a and 2b, and thus high sensitization can be suitably obtained. Furthermore, number of the type of the secondary antibody to be used is not limited to 3, and it may be either increased or decreased depending on the necessity of sensitization, and it may be 2 types, for example. The same shall apply to the primary antibody.

### [Fourth embodiment]

Because only the immunostaining step of the first embodiment is modified in the fourth embodiment, explanations for other steps are omitted, and only the immunostaining step is explained hereinbelow.

### <First reaction step>

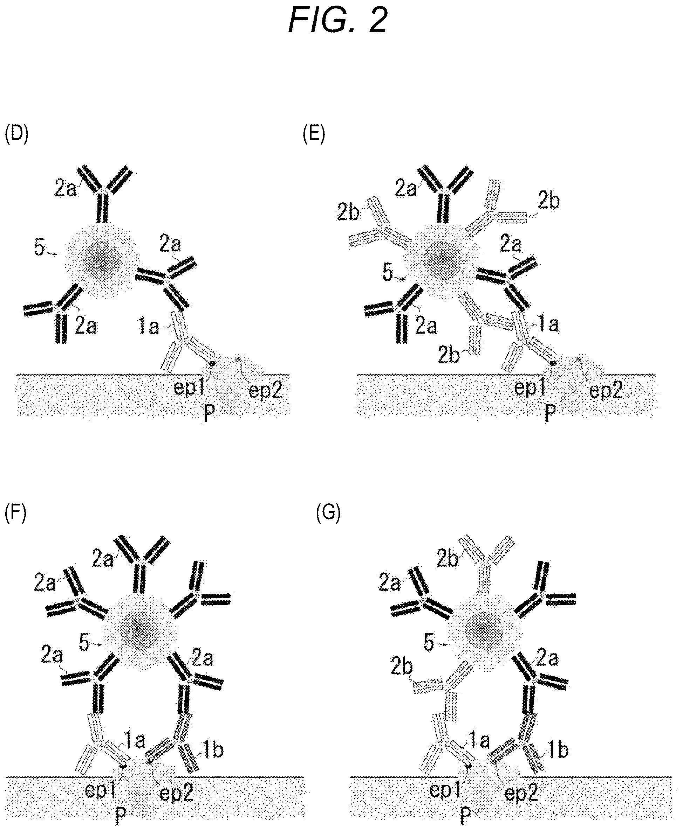

As it is shown in Fig. 2(D), a solution of the primary antibody 1a as a free monoclonal antibody is applied on a sample slide, and an antigen and antibody reaction is allowed to occur. Accordingly, the primary antibody 1a binds only to the epitope ep1 that is included in the antigen P of the sample slide.

### <Second reaction step>

As illustrated in Fig. 2(D), the second reaction step in which Fc region (part or whole) of the primary antibody 1a or Fab region (part or whole) thereof not bound to the antigen P (that is, primary antibody part not contributing to the reaction with the antigen P) is bound with the secondary antibody 2a as a monoclonal antibody that is concentrated and directly bound in large number on a surface of the fluorescent nanoparticle 5 is carried out so that the fluorescent nanoparticle 5 is fixed on the antigen P.

### <Third reaction step>

Before the first reaction step or the second reaction step, the fluorescent nanoparticle 5 is bound with plural secondary antibodies 2a as it has been described above for the second antibody.

### (Action and effect of fourth embodiment)

Hereinbelow, the action and effect of the fourth embodiment are explained.

According to the fluorescent immunostaining method of the fourth embodiment, because the primary antibody 1a is a monoclonal antibody, binding of the primary antibody 1a can be achieved in a state in which the specificity for the antigen P is maintained, in particular. In addition, since the secondary antibody 2a is concentrated and directly bound on a surface of the fluorescent nanoparticle 5, compared to the secondary antibody in free state, the secondary antibody 2a can be bound to the primary antibody 1a at higher probability, and, based on this binding, the fluorescent nanoparticle 5 can be bound with the secondary antibody, and thus high sensitization can be suitably obtained.

Furthermore, depending on the characteristics of the secondary antibody (binding affinity for primary antibody or the like), binding density of the secondary antibody 2a on a surface of the fluorescent nanoparticle 5 and average particle size of the fluorescent nanoparticle 5 (that is, number of secondary antibody per particle) can be modified.

### [Fifth embodiment]

Because only the immunostaining step of the first embodiment is modified in the fifth embodiment, explanations for other steps are omitted, and only the immunostaining step is explained hereinbelow.

### <First reaction step>

As it is shown in Fig. 2(E), a solution of the primary antibody 1a as a free monoclonal antibody is applied on a sample slide, and an antigen and antibody reaction is allowed to occur. Accordingly, the primary antibody 1a binds only to the epitope ep1 that is included in the antigen P of the sample slide.

### <Second reaction step>

As illustrated in Fig. 2(E), the second reaction step in which Fc region (part or whole) of the primary antibody 1a or Fab region (part or whole) thereof not bound to the antigen P (that is, primary antibody part not contributing to the reaction with the antigen P) is bound with the secondary antibodies 2a and 2b as a polyclonal antibody directly bound in plurality on a surface of the fluorescent nanoparticle 5 is carried out so that the secondary antibodies 2a and 2b and the fluorescent nanoparticle 5 are fixed on the antigen P.

### <Third reaction step>

Before the first reaction step or the second reaction step, plural secondary antibodies 2a and 2b are bound to the fluorescent nanoparticle 5 as it has been described above with regard to the second antibody.

### (Action and effect of fifth embodiment)

Hereinbelow, the action and effect of the fifth embodiment are explained.

According to the fluorescent immunostaining method of the fifth embodiment, because the primary antibody 1a is a monoclonal antibody, binding of the primary antibody can be achieved in a state in which the specificity for the antigen P is maintained, in particular. In addition, since the secondary antibodies 2a and 2b as a polyclonal antibody are concentrated and directly bound in large number on a surface of the fluorescent nanoparticle 5, compared to a case in which monoclonal antibodies are present in large number in concentrated state, linking and fixing is achieved at multiple different sites of a primary antibody (see, Fig. 2(E)), and at even higher probability and specificity, the secondary antibody 2a and the fluorescent nanoparticle 5 can be bound and fixed on the primary antibody, and thus high sensitization can be suitably obtained.

### [Sixth embodiment]

Because only the immunostaining step of the first embodiment is modified in the sixth embodiment, explanations for other steps are omitted, and only the immunostaining step is explained hereinbelow.

### <First reaction step>

As it is shown in Fig. 2(F), a solution of the primary antibodies 1a and 1b as a free polyclonal antibody is applied on a sample slide, and an antigen and antibody reaction is allowed to occur. Accordingly, each of the primary antibodies 1a and 1b binds to plural epitopes ep1 and ep2 that are included in the antigen P of the sample slide.

### <Second reaction step>

As illustrated in Fig. 2(F), the second reaction step in which Fc region (part or whole) of the primary antibodies 1a and 1b, or Fab region (part or whole) thereof (that is, primary antibody part not contributing to the reaction with the antigen P) is bound with the secondary antibody 2a as a monoclonal antibody that is concentrated and directly bound in large number on a surface of the fluorescent nanoparticle 5 is carried out so that 2 or more of the secondary antibody 2a and the fluorescent nanoparticle 5 are fixed on the antigen P.

### <Third reaction step>

Before the first reaction step or the second reaction step, plural secondary antibodies 2a are bound to the fluorescent nanoparticle 5 as it has been described above with regard to the second antibody.

### (Action and effect of sixth embodiment)

Hereinbelow, the action and effect of the sixth embodiment are explained.

According to the fluorescent immunostaining method of the sixth embodiment, because the primary antibodies 1a and 1b as a polyclonal antibody bind to epitopes ep1 and ep2 of the antigen P, and plural secondary antibodies 2a as a monoclonal antibody concentrated and directly bound on a surface of the fluorescent nanoparticle 5 bind to part or whole of the Fc region or Fab region commonly present in the primary antibodies 1a and 1b, regardless of the type of the primary antibody, and also at multiple sites, the fluorescent nanoparticle is firmly fixed on the antigen P so that easy detection of the antigen P is achieved.

### [Seventh embodiment]

Because only the immunostaining step of the first embodiment is modified in the seventh embodiment, explanations for other steps are omitted, and only the immunostaining step is explained hereinbelow.

### <First reaction step>

As it is shown in Fig. 2(G), a solution of the primary antibodies 1a and 1b as a free polyclonal antibody is applied on a sample slide, and an antigen and antibody reaction is allowed to occur. Accordingly, the primary antibodies 1a and 1b bind to plural epitopes ep1 and ep2 that are included in the antigen P of the sample slide.

### <Second reaction step>

As illustrated in Fig. 2(G), the second reaction step in which Fc region (part or whole) of the primary antibodies 1a and 1b, or Fab region (part or whole) thereof not bound to the antigen P (that is, primary antibody part not contributing to the reaction with the antigen P) is bound with the secondary antibodies 2a and 2b as plural polyclonal antibodies directly bound to the fluorescent nanoparticle 5 is carried out so that 2 or more of the secondary antibodies 2a and 2b and fluorescent nanoparticle 5 are bound and fixed to one antigen P.

### <Third reaction step>

Before the first reaction step or the second reaction step, plural secondary antibodies 2a and 2b are bound to the fluorescent nanoparticle 5 as it has been described before.

### (Action and effect of seventh embodiment)

Hereinbelow, the action and effect of the seventh embodiment are explained.

According to the fluorescent immunostaining method of the seventh embodiment, because each of the primary antibodies 1a and 1b as a polyclonal antibody binds to the antigen P and the secondary antibodies 2a and 2b concentrated and directly bound on a surface of the fluorescent nanoparticle 5 bind to part or whole of the Fc region of the primary antibodies 1a and 1b or Fab region (part or whole) thereof, each at different site, the fluorescent nanoparticle can be firmly fixed, in a state with high specificity, in particular, on the antigen. Furthermore, number of the type of the primary antibody and the secondary antibody to be used is not limited to 2, and it may be either increased or decreased depending on the necessity of sensitization, and it may be 3 types, for example.

### [Eighth embodiment]

Because only the immunostaining step of the first embodiment is modified in the eighth embodiment, explanations for other steps are omitted, and only the immunostaining step is explained hereinbelow.

### <First reaction step>

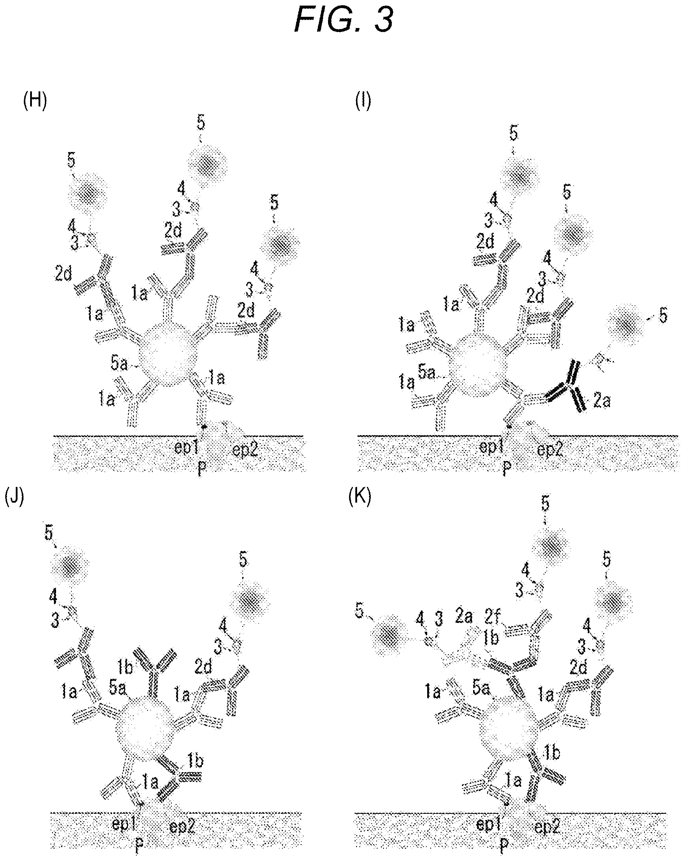

As it is shown in Fig. 3(H), a particle dispersion of the non-fluorescent nanoparticle 5a in which the primary antibody 1a as a monoclonal antibody is concentrated and bound in large number on a surface of the particle is applied on a sample slide, and an antigen and antibody reaction is allowed to occur. Accordingly, together with the non-fluorescent nanoparticle 5a, monoclonal antibody 1a binds to plural epitopes ep1 included in the antigen P of the sample slide.

Furthermore, use amount of the non-fluorescent nanoparticle 5a in which primary antibody is bound on a surface of the particle is mainly determined based on the binding density of the primary antibody on a surface of the non-fluorescent nanoparticle 5a, binding affinity of the primary antibody for the antigen, and average particle size of the non-fluorescent nanoparticle 5a, and it is not particularly limited as long as it is a concentration allowing the binding of the primary antibody to an antigen. For example, the non-fluorescent nanoparticle 5a can be used after being dispersed in a buffer like PBS such that the concentration of the primary antibody present on a surface of the non-fluorescent nanoparticle 5a is identical to the concentration of the free primary antibody which has been explained above in the first embodiment.

Compared to a primary antibody in which, between the antigen and fluorescent nanoparticle, a monoclonal primary antibody and a secondary antibody, biotin-avidin binding are present, mole number (or number) and molar concentration of the primary antibody on a surface of the non-fluorescent nanoparticle are preferably set at 10 times or higher, although it may vary depending on antibody titer.

Furthermore, the binding of a monoclonal antibody 1a to the antigen can be carried out at the same conditions as common primary antibody reaction, and it can be carried out by incubation for 4 to 12 hours at 1 to 10°C, for example.

### <Second reaction step>

As illustrated in Fig. 3(H), the second reaction step in which Fab region (part or whole) of the primary antibody 1a that is concentrated in large number on a surface of the non-fluorescent nanoparticle 5a fixed on the antigen P, or Fc region (part or whole) thereof not bound to the antigen P (that is, primary antibody part not contributing to the reaction with the antigen P) is bound with the secondary antibody 2d as plural free polyclonal antibodies is carried out.

### <Third reaction step>

By way of binding between the biotin 3 and the streptavidin 4, the fluorescent nanoparticle 5 is fixed on the secondary antibody 2d. As a result, 2 or more secondary antibody 2d and fluorescent nanoparticle 5 are bound and fixed to one antigen P.

### (Action and effect of eighth embodiment)

Hereinbelow, the action and effect of the eighth embodiment are explained.

According to the fluorescent immunostaining method of the eighth embodiment, because plural primary antibodies 1a are present in concentrated manner on a surface of the non-fluorescent nanoparticle 5a, compared to a case in which the primary antibody 1a is in a free state, binding of the primary antibody 1a to the antigen P can be obtained at higher probability. Furthermore, for one antigen P, the primary antibody 1a as a monoclonal antibody binds only at one site, and thus the specificity is high. In addition, plural secondary antibodies 2d bind to plural primary antibodies 1a that are present in concentrated manner on a surface of the non-fluorescent nanoparticle 5a but not reacted with the antigen P, and the fluorescent nanoparticle 5 binds to each of those the secondary antibody 2d, high sensitization can be achieved with very favorable efficiency while the specificity is suitably maintained. It is particularly advantageous in that, by adjusting the amount of the primary antibody 1a, the secondary antibody 2d, or the fluorescent nanoparticle 5 to be used, the detection sensitivity can be controlled within a broad range and a broader dynamic range of the sensitivity is obtained.

### [Ninth embodiment]

Because only the immunostaining step of the first embodiment is modified in the ninth embodiment, explanations for other steps are omitted, and only the immunostaining step is explained hereinbelow.

### <First reaction step>

As it is shown in Fig. 3(I), a particle dispersion of the non-fluorescent nanoparticle 5a in which the primary antibody 1a as a monoclonal antibody is concentrated and bound in large number on a surface of the particle is applied on a sample slide, and an antigen and antibody reaction is allowed to occur. Accordingly, the primary antibody 1a binds to plural epitopes ep1 included in the antigen P of the sample slide. In addition, the use amount of the non-fluorescent nanoparticle 5b having the primary antibody 1a concentrated and bound in large number on a surface of the particle is the same as that of the eighth embodiment.

### <Second reaction step>

As illustrated in Fig. 3(I), Fc region (part or whole) of plural primary antibodies 1a that are not bound to the antigen P, which are present on a surface of the non-fluorescent nanoparticle 5b fixed on the antigen P, or Fab region (part or whole) thereof (that is, primary antibody part not contributing to the reaction with the antigen P) is bound with the secondary antibodies 2a and 2d as plural free polyclonal antibodies.

### <Third reaction step>

By way of binding between the biotin 3 and the streptavidin 4, the fluorescent nanoparticle 5 is fixed on the secondary antibodies 2a and 2d. As a result, 2 or more secondary antibody 2d and fluorescent nanoparticle 5 are bound and fixed to one antigen P.

### (Action and effect of ninth embodiment)

Hereinbelow, the action and effect of the ninth embodiment are explained.

According to the fluorescent immunostaining method of the ninth embodiment, because plural primary antibodies 1a are present in concentrated manner on a surface of the non-fluorescent nanoparticle 5b, compared to a case in which the primary antibody 1a is in a free state, binding of the primary antibody 1a to the antigen P can be obtained at higher probability. Furthermore, for one antigen P, the primary antibody 1a as a monoclonal antibody binds only at one site, and thus the specificity is high. In addition, the secondary antibodies 2a and 2d as a polyclonal antibody bind to multiple sites of plural primary antibodies 1a present on a surface of the non-fluorescent nanoparticle 5a and not reacted with the antigen P, and also the fluorescent nanoparticle 5a binds to those secondary antibodies 2a and 2d, and thus an increased binding number of the secondary antibody is obtained and high sensitization can be achieved with favorable efficiency compared to a case of using a monoclonal antibody as the secondary antibody. As a result, a broader dynamic range of the sensitivity is obtained, and also, by adjusting the amount of the primary antibody, secondary antibody, or fluorescent nanoparticle to be used within the broad dynamic range, the detection sensitivity can be controlled suitably in broad range.

### [Tenth embodiment]

Because only the immunostaining step of the first embodiment is modified in the tenth embodiment, explanations for other steps are omitted, and only the immunostaining step is explained hereinbelow.

### <First reaction step>

As it is shown in Fig. 3(J), a particle dispersion of the non-fluorescent nanoparticle 5a in which the primary antibodies 1a and 1b as a polyclonal antibody are concentrated and bound in large number on a surface of the particle is applied on a sample slide, and an antigen and antibody reaction is allowed to occur. Accordingly, the primary antibodies 1a and 1b bind to plural epitopes ep1 and ep2 included in the antigen P of the sample slide. In addition, the use amount of the non-fluorescent nanoparticle 5a having the primary antibodies 1a and 1b concentrated and bound in large number on a surface of the particle is the same as that of the eighth embodiment.

### <Second reaction step>

As illustrated in Fig. 3(J), Fc region (part or whole) of plural primary antibodies 1a and 1b present on a surface of the non-fluorescent nanoparticle 5a fixed on the antigen P, or Fab region (part or whole) thereof (that is, primary antibody part not contributing to the reaction with the antigen P) is bound with plural secondary antibodies 2d as a free monoclonal antibody.

### <Third reaction step>

By way of binding between the biotin 3 and the streptavidin 4, the fluorescent nanoparticle 5 is fixed on the secondary antibody 2d. As a result, 2 or more secondary antibody 2d and fluorescent nanoparticle 5 are bound and fixed to one antigen P.

### (Action and effect of tenth embodiment)

Hereinbelow, the action and effect of the tenth embodiment are explained.

According to the fluorescent immunostaining method of the tenth embodiment, because plural primary antibodies 1a and 1b are present on a surface of the non-fluorescent nanoparticle 5a, compared to a case in which the primary antibodies 1a and 1b are in a free state, binding of the primary antibodies 1a and 1b to the antigen P can be obtained at higher probability. Furthermore, because the primary antibodies 1a and 1b are a polyclonal antibody, the non-fluorescent nanoparticle is more firmly fixed on the antigen P compared to the case of the ninth embodiment (case in which a monoclonal antibody is fixed on a surface of the non-fluorescent nanoparticle 5a). Thus, by having high specificity, high sensitization can be suitably achieved.

### [Eleventh embodiment]

Because only the immunostaining step of the first embodiment is modified in the eleventh embodiment, explanations for other steps are omitted, and only the immunostaining step is explained hereinbelow.

### <First reaction step>

As it is shown in Fig. 3(K), a particle dispersion of the non-fluorescent nanoparticle 5a in which plural polyclonal antibodies 1a and 1b are bound on a surface of the particle is applied on a sample slide, and an antigen and antibody reaction is allowed to occur. Accordingly, the primary antibodies 1a and 1b as a polyclonal antibody bind to plural epitopes ep1 and ep2 included in the antigen P of the sample slide. In addition, the use amount of the non-fluorescent nanoparticle 5a having the primary antibodies 1a and 1b concentrated and bound in large number on a surface of the particle is the same as that of the eighth embodiment.

### <Second reaction step>

As illustrated in Fig. 3(K), Fc region (part or whole) of plural primary antibodies 1a, which have not bound to the antigen P and are present on a surface of the non-fluorescent nanoparticle 5a fixed on the antigen P, or Fab region (part or whole) thereof (that is, primary antibody part not contributing to the reaction with the antigen P) is bound with plural secondary antibodies 2a, 2d, and 2f as a free polyclonal antibody.

### <Third reaction step>

By way of binding between the biotin 3 and the streptavidin 4, the fluorescent nanoparticle 5 is fixed on the secondary antibodies 2a, 2d, and 2f. As a result, 2 or more secondary antibodies 2a, 2d, and 2f and fluorescent nanoparticle 5 are bound and fixed to one antigen P.

### (Action and effect of eleventh embodiment)

Hereinbelow, the action and effect of the eleventh embodiment are explained.

According to the fluorescent immunostaining method of the eleventh embodiment, because plural primary antibodies 1a and 1b are present on a surface of the non-fluorescent nanoparticle 5a, compared to a case in which the primary antibodies 1a and 1b are in a free state, binding of the primary antibody to the antigen P can be obtained at higher probability. Furthermore, because the primary antibodies 1a and 1b are a polyclonal antibody, the non-fluorescent nanoparticle 5a is more firmly fixed on the antigen P compared to a case in which a monoclonal antibody is fixed on a surface of the non-fluorescent nanoparticle 5a. Thus, higher specificity is obtained. Furthermore, because the secondary antibodies 2a, 2d, and 2f as a free polyclonal antibody bind to two or more of the part or whole of Fc region or Fab region of the plural primary antibodies 1a and 1b present on a surface of the non-fluorescent nanoparticle 5a fixed on the antigen and also the fluorescent nanoparticle 5 binds to the secondary antibody, the binding number of the secondary antibody and fluorescent nanoparticle is higher and the dynamic range of detection is broader due to higher detection sensitivity than a case in which the secondary antibody is a monoclonal antibody.

### [Twelfth embodiment]

Because only the immunostaining step of the first embodiment is modified in the twelfth embodiment, explanations for other steps are omitted, and only the immunostaining step is explained hereinbelow.

### <First reaction step>

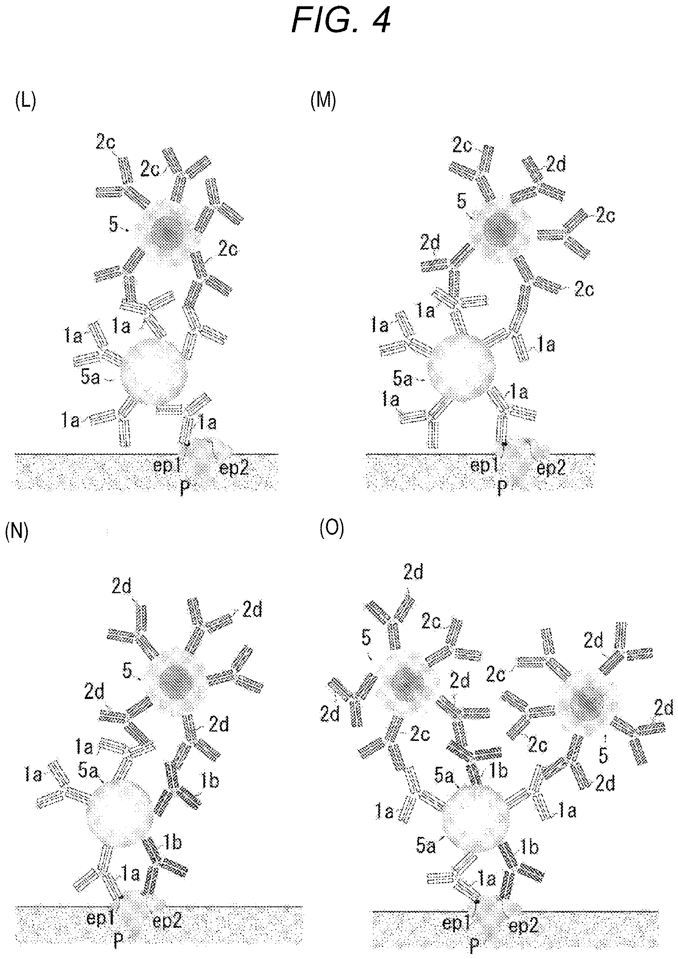

As it is shown in Fig. 4(L), a particle dispersion of the non-fluorescent nanoparticle 5a in which plural monoclonal antibodies 1a are bound on a surface of the particle is applied on a sample slide, and an antigen and antibody reaction is allowed to occur. Accordingly, the primary antibody 1a as a monoclonal antibody binds to epitope ep1 included in the antigen P of the sample slide. In addition, the use amount of the non-fluorescent nanoparticle 5a having the primary antibody 1a concentrated and bound in large number on a surface of the particle is the same as that of the eighth embodiment.

### <Second reaction step>

As illustrated in Fig. 4(L), Fc region (part or whole) of plural primary antibodies 1a, which are present on a surface of the non-fluorescent nanoparticle 5a fixed to one antigen P, or Fab region (part or whole) thereof (that is, primary antibody part not contributing to the reaction with the antigen P) is bound with plural secondary antibodies 2c as a monoclonal antibody bound in large number on a surface of the fluorescent nanoparticle 5, and, by way of the binding of 2 or more secondary antibody 2c to one antigen P, one or more fluorescent nanoparticles 5 are fixed.

### <Third reaction step>

Before the first reaction step or the second reaction step, plural secondary antibodies 2c are bound to the fluorescent nanoparticle 5 as it has been described before.

### (Action and effect of twelfth embodiment)

Hereinbelow, the action and effect of the twelfth embodiment are explained.

According to the fluorescent immunostaining method of the twelfth embodiment, because the primary antibody 1a is a monoclonal antibody, the primary antibody binding is obtained while the specificity for the antigen is maintained, in particular. Because plural primary antibodies 1a are present in concentrated manner on a surface of the non-fluorescent nanoparticle 5a, compared to a case in which the primary antibody 1a is in a free state, binding of the primary antibody to the antigen P can be obtained at higher probability. Furthermore, because the secondary antibody 2c, which is bound in large number on a surface of the fluorescent nanoparticle 5, binds to plural primary antibodies 1a on a surface of the non-fluorescent nanoparticle 5a which have not reacted with the antigen P, the secondary antibody 2c can be bound to the primary antibody 1a at high probability, and thus high sensitization can be suitably achieved.

### [Thirteenth embodiment]

Because only the immunostaining step of the first embodiment is modified in the thirteenth embodiment, explanations for other steps are omitted, and only the immunostaining step is explained hereinbelow.

### <First reaction step>

As illustrated in Fig. 4(M), a particle dispersion of the non-fluorescent nanoparticle 5a in which the monoclonal antibody 1a is concentrated and bound in large number on a surface of the particle is applied on a sample slide, and an antigen and antibody reaction is allowed to occur. Accordingly, the monoclonal antibody 1a (primary antibody) binds to epitope ep1 included in the antigen P of the sample slide. In addition, the use amount of the non-fluorescent nanoparticle 5a having the primary antibody 1a concentrated and bound in large number on a surface of the particle is the same as that of the eighth embodiment.

### <Second reaction step>

As illustrated in Fig. 4(M), Fc region (part or whole) of plural primary antibodies 1a, which are present on a surface of the non-fluorescent nanoparticle 5a fixed on the antigen P and not bound to the antigen P, or Fab region (part or whole) thereof (that is, primary antibody part not contributing to the reaction with the antigen P) is bound with plural secondary antibodies 2d and 2c as a polyclonal antibody bound in large number on a surface of the fluorescent nanoparticle 5. Accordingly, by way of the binding of 2 or more of the secondary antibody 1a to one antigen P, one or more fluorescent nanoparticles 5 are fixed.

### <Third reaction step>

Before the first reaction step or the second reaction step, plural secondary antibodies 2c and 2d are bound to the fluorescent nanoparticle 5 as it has been described before.

### (Action and effect of thirteenth embodiment)

Hereinbelow, the action and effect of the thirteenth embodiment are explained.

According to the fluorescent immunostaining method of the thirteenth embodiment, because the primary antibody 1a is a monoclonal antibody, binding of the primary antibody 1a is obtained while the specificity for the antigen P is maintained, in particular. Because plural primary antibodies 1a are present on a surface of the non-fluorescent nanoparticle 5a, compared to a case in which the primary antibody 1a is in a free state, binding of the primary antibody 1a to the antigen P can be obtained at higher probability. Furthermore, because the polyclonal antibodies 2c and 2d, which are concentrated and bound in large number on a surface of the fluorescent nanoparticle 5, bind, as a secondary antibody, to plural primary antibodies 1a on a surface of the non-fluorescent nanoparticle 5a which have not reacted with the antigen P, the secondary antibody can bind to multiples sites of the primary antibody 1a at high probability, and thus high sensitization can be suitably achieved.

### [Fourteenth embodiment]

Because only the immunostaining step of the first embodiment is modified in the fourteenth embodiment, explanations for other steps are omitted, and only the immunostaining step is explained hereinbelow.

### <First reaction step>

As illustrated in Fig. 4(N), a particle dispersion of the non-fluorescent nanoparticle 5a in which the primary antibodies 1a and 1b as a polyclonal antibody are concentrated and bound in large number on a surface of the particle is applied on a sample slide, and an antigen and antibody reaction is allowed to occur. Accordingly, the primary antibodies 1a and 1b bind to plural epitopes ep1 and ep2 included in the antigen P of the sample slide, and thus the non-fluorescent nanoparticle 5a is fixed on the antigen P. In addition, the use amount of the non-fluorescent nanoparticle 5a having the primary antibodies 1a and 1b bound on a surface of the particle is the same as that of the eighth embodiment.

### <Second reaction step>

As illustrated in Fig. 4(N), Fc region (part or whole) of plural primary antibodies 1a and 1b, which are present on a surface of the non-fluorescent nanoparticle 5a fixed on the antigen P and not bound to the antigen P, or Fab region (part or whole) thereof (that is, primary antibody part not contributing to the reaction with the antigen P) is bound with plural secondary antibodies 2d as a monoclonal antibody bound in large number on a surface of the fluorescent nanoparticle 5, and, by way of the binding of 2 or more antibodies to one antigen, one or more fluorescent nanoparticles are fixed.

Compared to a secondary antibody in which, between the antigen and fluorescent nanoparticle, monoclonal primary antibody and secondary antibody, biotin-avidin binding are present, mole number (or number) and molar concentration of the secondary antibody on a surface of the fluorescent nanoparticle are preferably set at 10 times or higher, although it may vary depending on antibody titer (ditto for the followings).

### <Third reaction step>

Before the first reaction step or the second reaction step, plural secondary antibodies 2d are bound to the fluorescent nanoparticle 5 as it has been described before.

### (Action and effect of fourteenth embodiment)

Hereinbelow, the action and effect of the fourteenth embodiment are explained.

According to the fluorescent immunostaining method of the fourteenth embodiment, because plural primary antibodies 1a and 1b are present on a surface of the non-fluorescent nanoparticle 5a, compared to a case in which the primary antibody is in a free state, binding of the primary antibody 1a to the antigen P can be obtained at higher probability. Furthermore, because plural primary antibodies 1a and 1b are a polyclonal antibody, the non-fluorescent nanoparticle 5a is more firmly fixed on the antigen P than a case in which a monoclonal antibody is fixed on a surface of the non-fluorescent nanoparticle 5a, and thus higher specificity is obtained.

In addition, to a part commonly present in plural primary antibodies 1a and 1b on a surface of the non-fluorescent nanoparticle 5a, which have not reacted with the antigen P (e.g., part of Fc region), the monoclonal antibody 2d bound in large number on a surface of the fluorescent nanoparticle 5 binds, and thus the binding can be achieved regardless of the type of the primary antibody 1a, and also the secondary antibodies 2c and 2d can bind to the primary antibody 1a at high probability. Accordingly, high sensitization can be suitably obtained.

### [Fifteenth embodiment]

Because only the immunostaining step of the first embodiment is modified in the fifteenth embodiment, explanations for other steps are omitted, and only the immunostaining step is explained hereinbelow.

### <First reaction step>

As illustrated in Fig. 4(O), a particle dispersion of the non-fluorescent nanoparticle 5a in which the primary antibodies 1a and 1b as a polyclonal antibody are concentrated and bound in large number on a surface of the particle is applied on a sample slide, and an antigen and antibody reaction is allowed to occur. Accordingly, each of the primary antibodies 1a and 1b binds to plural epitopes ep1 and ep2 included in the antigen P of the sample slide. In addition, the use amount of the non-fluorescent nanoparticle 5a having the primary antibodies 1a and 1b bound on a surface of the particle is the same as that of the eighth embodiment.

### <Second reaction step>

As illustrated in Fig. 4(O), Fc region (part or whole) of the primary antibodies 1a and 1b, which are present on a surface of the non-fluorescent nanoparticle 5a fixed on the antigen P, or Fab region (part or whole) thereof (that is, primary antibody part not contributing to the reaction with the antigen P) is bound with polyclonal antibodies 2d and 2c (secondary antibody) that are bound in large number on a surface of the fluorescent nanoparticle 5, and, by way of the binding of 2 or more secondary antibodies 2c and 2d to one antigen P, one or more fluorescent nanoparticles 5 are fixed.

### <Third reaction step>

Before the first reaction step or the second reaction step, plural secondary antibodies 2c and 2d are bound to the fluorescent nanoparticle 5 as it has been described before.

### (Action and effect of fifteenth embodiment)

Hereinbelow, the action and effect of the fifteenth embodiment are explained.

According to the fluorescent immunostaining method of the fifteenth embodiment, because plural primary antibodies 1a and 1b are present in concentrated manner on a surface of the non-fluorescent nanoparticle 5a, compared to a case of a free primary antibody, binding of the primary antibodies 1a and 1b to the antigen P can be obtained at higher probability. Furthermore, because the primary antibodies 1a and 1b on a surface of the non-fluorescent nanoparticle 5a are a polyclonal antibody, the non-fluorescent nanoparticle 5a is more firmly fixed on the antigen P than a case in which a monoclonal antibody is fixed on a surface of the non-fluorescent nanoparticle 5a, and thus higher specificity is obtained.

In addition, with regard to plural primary antibodies 1a and 1b present on a surface of the non-fluorescent nanoparticle 5a which have not reacted with the antigen P, the polyclonal antibody bound in large number on a surface of the fluorescent nanoparticle 5 binds, as a secondary antibody, to different binding sites of the primary antibodies 1a and 1b (e.g., whole Fc region and part of Fab region), and thus the secondary antibody can bind firmly to the primary antibody at high probability. Accordingly, high sensitization can be suitably obtained.

### Examples

### [Preparation Example 1] (Production of fluorescent nanoparticle (average particle size of 150 nm))

Production of the fluorescent nanoparticle (average particle size of 150 nm) was carried out according to the followings steps (1-1) to (1-6).
Step (1-1): As a fluorescent dye, 14.4 mg of Sulforhodamine 101 (manufactured by Sigma-Aldrich, Inc.), which is a red color emitting dye, is added and dissolved in 22 mL of distilled water.
   After that, 2 mL of 5% aqueous solution of Emulsion 430 (registered trademark) (polyoxyethylene oleyl ether, manufactured by Kao Corporation) as an emulsifier for emulsion polymerization were added to the above solution.
Step (1-2): While stirring the solution on a hot stirrer, the temperature was raised to 70°C. Thereafter, 0.65 g of NIKALAC MX-035 (manufactured by NIPPON CARBIDE INDUSTRIES CO., INC.) as a melamine resin raw material was added to the solution.
Step (1-3): Furthermore, as a surfactant, 1000 µL of 10% aqueous solution of dodecylbenzene sulfonate (manufactured by KANTO CHEMICAL CO., INC.) were added to the solution followed by stirring under heating at 70°C for 50 minutes.
Step (1-4): After that, the temperature was raised to 90°C, and stirring under heating was carried out for 20 minutes.
Step (1-5): To remove impurities like excess resin materials or fluorescent dyes from a dispersion of the obtained melamine-based fluorescent nanoparticle, washing with pure water was carried out. Specifically, centrifuge was carried out at 20000 G for 15 minutes using a centrifugation device (Micro Refrigerated Centrifuge 3740 manufactured by KUBOTA Corporation), and after supernatant removal, re-dispersion was carried out according to ultrasonication by adding ultra-pure water.
Step (1-6): The fluorescent nanoparticle dispersion of Step (1-5) was subjected 5 times to washing based on centrifuge, supernatant removal, and re-dispersion with ultra-pure water. As the melamine resin itself contains many amino groups in the skeleton, the obtained melamine-based fluorescent nanoparticles are positively charged. Charge evaluation of the resin particle was carried out by resin component analysis based on NMR, IR, or the like, and also the zeta potential measurement. The nanoparticles are observed under a scanning electron microscope (SEM; S-800 manufactured by Hitachi (registered trademark)), and the average particle size and variation coefficient were calculated. Average particle size of the obtained fluorescent nanoparticle was 150 nm and the variation coefficient was 14.7%.

### [Preparation Example 2] <Production of fluorescent nanoparticle bound with polyclonal antibody> (Introduction of amino group to fluorescent nanoparticle)

By carrying out the following Steps (2-1) to (2-3) for the melamine-based fluorescent nanoparticle (average particle size of 150 nm) which has been prepared in Preparation Example 1, an amino group was introduced to the fluorescent nanoparticle.
Step (2-1): 0.1 mg of the above fluorescent nanoparticle was dispersed in 1 mL pure water to prepare a dispersion. Subsequently, 20 µL of tris(2-aminoethyl)amine (20 µL) were added to the dispersion and stirred stirring under heating at 70°C for 20 minutes.
Step (2-2): The reaction mixture was centrifuged at 10000 G for 60 minutes to remove the supernatant.
Step (2-3): Precipitates were dispersed by adding ethanol, and centrifuge was carried out again. Washing with ethanol and pure water was carried out according to the same order, one for each.

As a result of carrying out FT-IR measurement and XPS measurement for the obtained fluorescent nanoparticles that are introduced with an amino group, absorption derived from an amino group was observed, and thus introduction of the amino group was confirmed.

### (Introduction of maleimide group)

According to the following Steps (3-1) to (3-3), the melamine-based fluorescent nanoparticles modified with maleimide group were prepared.
Step (3-1): The fluorescent nanoparticles that are introduced with an amino group were dispersed in PBS (phosphate buffered physiological saline) containing 2 mM EDTA such that it is present at 3 nM. The dispersion was admixed with SM(PEG)₁₂ (manufactured by Thermo Fischer Scientific, succinimidyl-[(N-maleimidepropioamide)-dodecane ethylene glycol]ester) such that it has final concentration of 10 mM, and the mixture was reacted for 1 hour at room temperature.
Step (3-2): The mixture after the reaction was centrifuged at 10000 G for 20 minutes to remove the supernatant, and then added with PBS containing 2 mM EDTA to disperse the precipitates. The centrifuge was carried out again.
Step (3-3): By carrying out the washing 3 times according to the same order as above, fluorescent nanoparticles added with maleimide group by way of a PEG chain were obtained.

### (Binding between fluorescent nanoparticle and polyclonal antibody)

Step (4-1): 100 µg of "Biotin labeled anti rabbit IgG antibody (animal species: goat)" (product number: 426011, 426012, NICHIREI BIOSCIENCES INC.) were dissolved in PBS (100 µL). To the antibody solution, 1 M 2-Mercaptoehanol was added in an amount of 0.002 mL (0.2 × 10⁻⁵ mol), and the reaction was allowed to occur at room temperature for 30 minutes at pH 8.5 to carry out the antibody reduction.
Step (4-2): The reaction solution after Step (4-1) was provided to a gel filtration column, and by removing excess 2-mercaptoehtanol, a solution of the antibody (anti rabbit IgG polyclonal antibody) having SH group was obtained.

### (Binding between anti HER2 antibody having SH group and sulforhodamine-containing melamine nanoparticle modified with maleimide group)

Step (5-1): 0.01 µg of the melamine-based fluorescent nanoparticle modified with maleimide group, which has been obtained by Step (3-3), and 10 µg of the antibody (anti rabbit IgG polyclonal antibody) having SH group, which has been obtained by Step (4-2), were admixed with each other in 1 mL PBS, and the reaction for binding the two molecules was carried out for 1 hour at room temperature.
Step (5-2): 4 µL of 10 mM 2-mercaptoethanol were added to the reaction solution after Step (5-1) to terminate the binding reaction.
Step (5-3): The solution from Step (5-2) was centrifuged for 60 minutes at 10000 g to remove the supernatant. After that, PBS containing 2 mM EDTA was added thereto to disperse the precipitates, and the centrifuge was carried out again. Washing was carried out 3 times according to the same order. Finally, the resultant was dispersed in PBS (500 µL) and fluorescent nanoparticles that are bound with an anti rabbit IgG polyclonal antibody as a secondary antibody were obtained.

### [Preparation Example 3] <Production of fluorescent nanoparticle directly bound with monoclonal antibody>

Melamine-based fluorescent nanoparticle bound with an anti rabbit IgG antibody (monoclonal antibody) as a secondary antibody was prepared in the same manner as Preparation Example 2 except that "LO-RG-1" (anti rabbit IgG antibody manufactured by Funakoshi Co., Ltd.) is used instead of "Biotin labeled anti rabbit IgG antibody (animal species: goat)" used in Preparation Example 2.

### [Preparation Example 4] <Production of fluorescent nanoparticles bound with polyclonal antibody>

Melamine nanoparticles not containing any fluorophore (non-fluorescent nanoparticles) were prepared in the same manner as Preparation Example 1 except that fluorescent dyes of Preparation Example 1 are not used.

Furthermore, a non-fluorescent melamine nanoparticle bound with anti PD-L1 antibody "Anti-PD-Ll antibody (ab58810)" was prepared in the same manner as Preparation Example 2 except that, with regard to Preparation Example 2, the above non-fluorescent nanoparticle is used instead of the fluorescent nanoparticles, and "Anti-PD-L1 antibody (ab58810)" (animal species: rabbit, Abcam), which is a polyclonal antibody capable of binding to PD-L1, is used instead of "Biotin labeled anti rabbit IgG antibody (animal species: goat)". In this case, the binding density of a primary antibody on a surface of the "Biotin labeled anti rabbit IgG antibody (animal species: goat)" was about 1000 antibodies per particle and it corresponds to about 3.54 × 10¹⁴ (antibody/mm² of particle surface).

### (Determination of antibody binding density)

By carrying out BCA method for the non-fluorescent melamine nanoparticle bound with an antibody as prepared above, the amount of proteins bound on a surface of the particle (total antibody weight) was measured, and based on the weight molecular weight of the antibody used, mole number or number of the antibody fixed on a surface of the particle was calculated.

Meanwhile, by using the average particle size of the non-fluorescent nanoparticle (150 nm), the average particle surface area was calculated based on 4πr². Furthermore, by using a particle counter, the dispersion concentration (particles/µL) of the non-fluorescent nanoparticle was determined, and from the total volume of the dispersion (µL), total number (particles) of the non-fluorescent nanoparticle was calculated. By multiplying the average particle surface area (mm2/particle) by the total number (particles) of the particles that are measured, total area (mm2) of the non-fluorescent nanoparticle was calculated. From the mole number of the antibody and total particle area (mm²), the antibody binding density (mole number of antibody/mm²) was calculated.

### [Preparation Example 5] <Production of non-fluorescent nanoparticles bound with monoclonal primary antibody>

Melamine nanoparticles not containing any fluorophore (non-fluorescent nanoparticle) were prepared in the same manner as Preparation Example 1 except that the fluorescent dyes of Preparation Example 1 are not used.

Furthermore, the non-fluorescent melamine nanoparticle bound with "PD-L1 (E1L3N (registered trademark) XP (registered trademark) Rabbit mAb" was prepared in the same manner as Preparation Example 3 except that, with regard to Preparation Example 3, the above non-fluorescent nanoparticle is used instead of the fluorescent nanoparticles, and PD-L1 (E1L3N (registered trademark) XP (registered trademark) Rabbit mAb (Cell Signaling Technology Japan, K.K.)" is used instead of "Biotin labeled anti rabbit IgG antibody (animal species: goat)" (product number: 426011, 426012, NICHIREI BIOSCIENCES INC.). As a result of measuring, according to the method for determining the antibody binding density of Preparation Example 4, the binding density of a primary antibody on a surface of a non-fluorescent nanoparticle in that case, it was found that about 1000 antibodies are bound to one particle and it corresponds to 3.54 × 10¹⁴ (antibody/mm² of particle surface).

### [Preparation Example 6] <Preparation of biotin-modified free polyclonal antibody (secondary antibody)>

Histofine "biotin labeled anti rabbit IgG antibody (animal species: goat)" (product number: 426011, 426012, NICHIREI BIOSCIENCES INC.) was purchased, and a solution of this antibody (containing 0.1% sodium azide) was provided to the following Step (6-1).
Step (6-1): The above antibody solution was purified by providing it to a desalting column "Zeba Desalt Spin Columns" (manufactured by Thermo Fisher Scientific Inc., Cat. #89882). The absorption of the desalted reaction solution at a wavelength of 300 nm was measured using a spectrophotometer ("F-7000", manufactured by Hitachi, Ltd.) and the amount of proteins contained in the reaction solution was calculated. The reaction solution was adjusted with a 50 mM Tris solution to a concentration of 250 µg/mL, and the resulting solution was used as a solution of a biotin-modified free polyclonal antibody (secondary antibody).

### [Preparation Example 7] <Production of biotin-modified free monoclonal antibody (secondary antibody)>

Step (7-1): 50 µg of "LO-RG-1" (anti rabbit IgG antibody manufactured by Funakoshi Co., Ltd.) were dissolved in 50 mM Tris solution.
Step (7-2): A DTT (dithiothreitol) solution was mixed with the solution to a final concentration of 3 mM Thereafter, the resulting solution was allowed to react at 37°C for 30 minutes.
Step (7-3): After that, DTT-reduced secondary antibody was purified using a desalting column.
Step (7-4): Of the whole amount of the thus-purified antibody, 200 µL was dissolved in a 50 mM Tris solution to obtain an antibody solution.
Step (7-5): Meanwhile, a linker reagent having a spacer length of 30 angstrom, "Biotin-PEG₆-NH-Mal" (manufactured by PurePEG, product No. 2461006-250), was adjusted with DMSO to a concentration of 0.4 mM
Step (7-6): This solution in an amount of 8.5 µL was added to the antibody solution, and the resultant was mixed and allowed to react with mixing at 37°C for 30 minutes.
Step (7-7): The resulting reaction solution was applied to a desalting column "Zeba Desalt Spin Columns" (manufactured by Thermo Fisher Scientific Inc., Cat. #89882) and purified. The absorption of the thus-desalted reaction solution at a wavelength of 300 nm was measured using a spectrophotometer ("F-7000", manufactured by Hitachi, Ltd.) and the amount of proteins contained in the reaction solution was calculated. The reaction solution was adjusted with a 50 mM Tris solution to a concentration of 250 µg/mL, and the resulting solution was used as a solution of biotin-modified free monoclonal antibody (secondary antibody).

### [Preparation Example 8] <Production of sulforhodamine-containing nanoparticle bound with streptavidin by way of linker)

### (Preparation of streaptavidin having SH group)

Production of streaptavidin having SH group was carried out as follows.
Step (8-1): 40 µL streptavidin adjusted to 1 mg/mL (manufactured by Wako Pure Chemical Industries, Ltd.) were reacted for 1 hour at room temperature with 70 µL of 2-iminothiolane (manufactured by Pierce) adjusted to 64 mg/mL. Namely, a thiol group was introduced to the amino group of streptavidin.
Step (8-2): The resulting streptavidin solution was desalted through a gel filtration column (Zaba Spin Desalting Columns: manufactured by Funakoshi Co., Ltd.) to obtain 0.04 mg of streptavidin having SH group.

### (Introduction of maleimide group to fluorescent nanoparticle)

Step (8-3): The fluorescent nanoparticles that are introduced with an amino group, which have been obtained in Step (2-3) of Preparation Example 2, were dispersed in PBS (phosphate buffered physiological saline) containing 2 mM EDTA such that it is present at 3 nM.
Step (8-4): The dispersion was admixed with SM(PEG)₁₂ (manufactured by Thermo Fischer Scientific, succinimidyl-[(N-maleimidepropioamide)-dodecane ethylene glycol]ester) as a linker to have final concentration of 10 mM, and the mixture was reacted for 1 hour at room temperature.
Step (8-5): The mixture after the reaction was centrifuged at 10000 G for 20 minutes to remove the supernatant, and then added with PBS containing 2 mM EDTA to disperse the precipitates. The centrifuge was carried out again. By carrying out the washing 3 times according to the same order as above, fluorescent nanoparticles added with maleimide group by way of a PEG chain were obtained.

### (Binding between fluorescent nanoparticle and streptavidin)

The fluorescent nanoparticles added with a maleimide group were dispersed in PBS containing 2 mM EDTA to 0.67 nM. This dispersion of fluorescent nanoparticles (740 µL) and the streptavidin introduced with SH group (0.04 mg) were admixed with each other, and allowed to react for 1 hour at room temperature. The reaction solution was added with 10 mM mercaptoethanol to terminate the reaction. The obtained solution was concentrated using a centrifuge filter, and by using a gel filtration column for purification, non-reacted antifluorescein antibody or the like are removed to obtain sulforhodamine-containing nanoparticle bound with streptavidin by way of linker. As a result of measuring the binding density of streptavidin on a surface of the fluorescent nanoparticle in that case, it was found that about 1000 streptavidins are bound to one particle and it corresponds to 3.54 × 10¹⁴ (streptavidin/mm² of particle surface).

### [Preparation Example 9]

### (Preparation of subject for staining (sample slide of PD-L1 culture cells))

According to a known method, a sample slide of PD-L1 positive culture cells fixed by paraffin was prepared.

Furthermore, for noise measurement, PD-L1 negative cells were cultured and fixed with paraffin to give a sample slide (hereinbelow, simply described as "sample slide (for noise measurement)). Furthermore, as a positive culture cell line, NCI-H460 (organism: homo sapiens, human/tissue: lung: pleural effusion/disorder: cancer: large cell lung cancer) was used, and as a negative culture cell line, NCI-H446 (organism: homo sapiens, human/tissue: lung: pleural effusion; originating from metastasis area/disorder: cancer: small cell lung cancer) manufactured by ATCC (registered trademark)) was used. Conditions for culture are as described below.

**[Table 2]**

| Cell name | Doubling time in 75-cm flask | Medium | Subculture |
|---|---|---|---|
| NCI-H460 | > 3days | RPMI (added with 10% FBS) | 0.25%Trypsin, 1:3-1:8 |
| NCI-H446 | > 3days | RPMI (added with 10% FBS) | 0.25%Trypsin, 1:3-1:6 |

Furthermore, the subculture was carried out according to generally known subculture. As shown in Table 2, 0.25% trypsin was used for cell surface washing during subculture. The ratio of the volume of fresh medium to addition amount of cell suspension to be added to the medium was set at 1 : 3 to 1 : 8 (or 1 : 3 to 1 : 6).

### (Formalin fixation and paraffin embedding)

Various culture cells which have been cultured as above were prepared to a sample slide based on general conditions for formalin fixation and paraffin embedding (FFPE), on the basis of the following Steps (A-1) to (A-8).
Step (A-1): Fresh tissue block of the PD-L1 culture cells, which is cut into 3 mm × 3 mm or so, was subjected to a 48-hour fixation treatment at room temperature by using formalin buffer "10% neutral buffered formalin solution" (by Wako Pure Chemical Industries, Ltd.).
Step (A-2): The tissue was washed for 1 hour with tap water.
Step (A-3): The tissue was immersed in 70%, 80%, and 95% ethanol, each for 45 minutes, and 100% ethanol was exchanged 3 times with fresh one, 1 hour for each, to have dehydration.
Step (A-4): The tissue was immersed in xylene, and the xylene was exchanged 2 times with fresh one, 1 hour for each.
Step (A-5): The tissue was immersed in paraffin, and the paraffin was exchanged 3 times with fresh one, 1 hour for each.
Step (A-6): The tissue was prepared in paraffin block.
Step (A-7): Using a microtome, the paraffin-embedded block was cut to a thin film of 6 µm, and allowed to float over distilled water at 40°C.
Step (A-8): The sample was transferred on a glass slide suitable for immunostaining, and, after drying overnight, it was kept at room temperature until use.

### [Comparative Example 1]

### (Immunostaining step)

### [Deparaffinization treatment step]

Step (1A): The sample slide was immersed in xylene for 10 minutes to remove paraffin in the sample slide and replace it with xylene. Xylene was exchanged with fresh one 3 times during the immersion.
Step (1B): The sample slide obtained after Step (1A) was immersed in ethanol for 5 minutes to replace the xylene in the sample slide with ethanol. Ethanol was exchanged with fresh one 3 times during the immersion.
Step (1C): The sample slide obtained after Step (1B) was immersed in distilled water for 5 minutes to replace the ethanol in the sample slide with distilled water. Distilled water was exchanged with fresh one 3 times during the immersion.

### [Activation treatment step]

Step (2A): The sample slide obtained after Step (1C) was immersed in 10 mM citrate buffer (pH 6.0) for 10 minutes to replace the water in the sample slide with citrate buffer.
Step (2B): The sample slide obtained after Step (2A) was subjected to an autoclave treatment (10 minutes at 121°C).
Step (2C): The sample slide obtained after Step (2B) was cooled to room temperature, and then, immersed in PBS for 5 minutes. PBS was exchanged with fresh one 3 times during the immersion.
Step (2D): PBS containing BSA at 1% by weight was added dropwise all over the sample slide obtained after Step (2C) followed by keeping for 30 minutes at room temperature.

### [First reaction step]

Step (3A): A solution containing non-fat dry milk at 5% w/v, 1 × TBS, and 0.1% Tween (registered trademark) 20 was prepared.
Step (3B): Using the solution prepared in Step (3A), "PD-L 1 (E1L3N (registered trademark) XP (registered trademark) Rabbit mAb" (animal species: rabbit, Cell Signaling Technology Japan, K.K.) as a primary antibody, which is an anti PD-L1 antibody capable of binding to human PD-L1, was diluted by 800 times. The resulting antibody solution (100 µL) was placed on top of a sample slide of PD-L1 culture cells followed by incubation overnight at 4°C.

### [Washing step 1]

Step (4A): The sample slide obtained after Step (3B) was immersed in PBS for 5 minutes for each. During the immersion, PBS exchange was carried out 3 times.

### [Second reaction step]

Step (5A): Using the solution prepared in Step (3A), the antibody solution of the biotin-modified free monoclonal antibody (secondary antibody) "LO-RG-1" (anti rabbit IgG antibody manufactured by Funakoshi Co., Ltd.) which has been prepared in Preparation Example 7 was diluted by 200 times. The resulting solution (100 µL) was placed on top of a sample slide of PD-L1 culture cells obtained after Step (4A), and the reaction was allowed to occur for 30 minutes at room temperature.

### [Washing step 2]

Step (6A): The sample slide obtained after Step (5A) was immersed in PBS for 5 minutes for each. During the immersion, PBS exchange was carried out 3 times.

### [Third reaction step]

Step (7A): Using PBS containing BSA at 1% by weight, the sulforhodamine-containing melamine-based fluorescent nanoparticles that are modified with streptavidin as prepared in Preparation Example 8 were diluted to 0.05 nM. Next, the dispersion obtained by this dilution was added dropwise all over the sample slide obtained after Step (2D) followed by keeping for 3 hours at room temperature.

### [Washing step 3]

Step (8A): Thereafter, the sample slide obtained after Step (5A) was immersed for 5 minutes in PBS for each. During the immersion, PBS exchange was carried out 3 times.

### [Treatment step for morphology observation]

Step (9A): The sample slide obtained after Step (8A) was fixed for 10 minutes using 4% neutral paraformaldehyde solution, and subjected to hematoxylin-eosin staining (HE staining). For the HE staining, the immunostained sample slide was stained for 15 minutes with Mayer's hematoxylin solution followed by hematoxylin staining. After that, the sample slide was washed with 45°C running water for 3 minutes. Subsequently, it was subjected to eosin staining for 1 minute with 1% eosin solution. After that, an operation of immersing it in pure ethanol for 5 minutes was repeated four times to perform washing and dehydration. Subsequently, an operation of immersing it in xylene for 5 minutes was repeated four times followed by clearing.
Step (9B): The whole sample slide obtained after Step (9A) was applied with "Aquatex (registered trademark)" (product number: 108562, manufactured by Merck Millipore), covered with a cover glass, and allowed to stand for 30 minutes or longer to seal the sample slide.

### [Observation step]

The sample slide obtained after a series of the above steps was illuminated with a prescribed excitation light to allow fluorescence to be emitted. The sample slide in this state was observed and photographed under a fluorescence microscope ("BX-53", manufactured by Olympus Corporation). Furthermore, the bright spot measurement was carried out based on ImageJ Find Maxima method.

The excitation light was set at 545 to 565 nm according to transmission through an optical filter. In addition, the wavelength range (nm) of the fluorescence to be observed was set at 570 to 590 nm according to transmission through an optical filter.

The conditions of the excitation wavelength in the microscope observation and image acquisition were set such that the irradiation energy in the vicinity of the center of the visual field is 900 W/cm² for excitation at 550 nm. In the image acquisition process, a photograph was taken by arbitrarily setting the exposure time such that the image brightness is not saturated (for example, the exposure time was set at 4,000 µs). Next, by using the photographed microscopic image taken by a fluorescence microscope or the like, when brightness is higher than predetermined threshold value, it is measured as bright spot, and number of the fluorescent nanoparticle or fluorescent signal intensity (signal (relative value) per cell was calculated. This signal value of Comparative Example 1 was taken as a standard (relative value = 1) for comparison with the examples that are described below.

Also for the sample slide (for noise measurement), the deparaffinization treatment step to observation step were carried out in the same manner as above to measure the signal value, which was then used as a standard (relative value = 1) for comparison of noise value of the examples that are described below.

Regarding the quantification property, it was "○". Furthermore, as a standard quantification property, when using PD-L1 culture cells with known expression amount, if the correlation (R²) with a fluorescent nanoparticle score of the expression amount (number of particles found by observation step that is carried out by the same process for each single cell) is R² ≥ 0.70, it is graded "○". If it is R² < 0.70, it is graded "×".

### [Example 1] (Primary antibody: free monoclonal antibody, secondary antibody: free polyclonal antibody)

In Example 1, except that the second reaction step of Comparative Example 1 is carried out as described below, other steps like the deparaffinization treatment step, the activation treatment step, the first reaction step, the third reaction step, the washing step, and the observation step were carried out in the same manner as Comparative Example 1.

### [Second reaction step]

Step (5A): By using the solution which has been prepared in Step (3A), the antibody solution of the biotin-modified free polyclonal antibody (secondary antibody) prepared in Preparation Example 6 was diluted 200 times, 100 µL of the resulting solution was applied on top of a sample slide of PD-L 1 culture cells obtained after Step (4A), and the reaction was allowed to occur for 30 minutes at room temperature.

### [Result]

As a result, the signal value compared to Comparative Example 1 was found to be 2. The noise value (relative value) indicates a value when the measured value of the sample slide (for noise measurement) of Comparative Example 1 is set at 1, and it was found to be 1.5. S/N ratio (relative value) was 1.33. Furthermore, the quantification property was "○".

### [Example 2] (Primary antibody: free polyclonal antibody, secondary antibody: free monoclonal antibody)

In Example 2, except that the first reaction step of Comparative Example 1 is carried out as described below, other steps like the deparaffinization treatment step, the activation treatment step, the second reaction step, the washing steps 1 to 3, the third reaction step, and the observation step were carried out in the same manner as Comparative Example 1.

### [First reaction step]

Step (3A): A solution containing non-fat dry milk at 5% w/v, 1 × TBS, and 0.1% Tween (registered trademark) 20 was prepared.
Step (3B): By using the solution which has been prepared in Step (3A), "Anti-PD-Ll antibody (ab58810)" (animal species: rabbit, Abcam), which is a polyclonal antibody capable of binding to human PD-L1, was diluted 200 times, and 100 µL of the resulting antibody solution was applied on top of a sample slide of PD-L1 culture cells and incubated overnight at 4°C.

Furthermore, the primary antibody was used after it has been adjusted to have the same mole number as the mole number of the primary antibody used in Comparative Example 1.

### [Result]

As a result, the signal value compared to Comparative Example 1 was found to be 2, and the noise value (relative value) was found to be 1. S/N ratio (relative value) was 2. Furthermore, the quantification property was "○". Result of Example 2 is shown in Table 3.

### [Example 3] (Primary antibody: free polyclonal antibody, secondary antibody: free polyclonal antibody)

In Example 3, except that the first reaction step and the second reaction step of Comparative Example 1 are carried out as described below, other steps including the deparaffinization treatment step, the activation treatment step, the washing steps 1 to 3, the third reaction step, and the observation step were carried out in the same manner as Comparative Example 1.

### [First reaction step]

Step (3A): A solution containing non-fat dry milk at 5% w/v, 1 × TBS, and 0.1% Tween (registered trademark) 20 was prepared.
Step (3B): By using the solution which has been prepared in Step (3A), "Anti-PD-Ll antibody (ab58810)" (animal species: rabbit, Abcam) was diluted 200 times, and 100 µL of the resulting antibody solution was applied on top of a sample slide of PD-L1 culture cells and incubated overnight at 4°C.

Furthermore, the primary antibody was used after it has been adjusted to have the same mole number as the mole number of the primary antibody used in Comparative Example 1.

### [Second reaction step]

Step (5A): By using the solution which has been prepared in Step (3A), the antibody solution of the biotin-modified free polyclonal antibody (secondary antibody) prepared in Preparation Example 6 was diluted 200 times, 100 µL of the resulting solution was applied on top of a sample slide of PD-L1 culture cells obtained after Step (4A), and the reaction was allowed to occur for 30 minutes at room temperature.

### [Result]

As a result of Example 3, the signal value compared to Comparative Example 1 was found to be 4, and the noise value (relative value) was found to be 3. S/N ratio (relative value) was 1.33. Furthermore, the quantification property was "○". Result of Example 3 is shown in Table 3.

### [Example 4] (Primary antibody: monoclonal antibody directly bound to non-fluorescent nanoparticle, secondary antibody: free monoclonal antibody)

In Example 3, except that the first reaction step of Comparative Example 1 is carried out as described below, other steps including the deparaffinization treatment step, the activation treatment step, the washing steps 1 to 3, the second reaction step, the third reaction step, and the observation step were carried out in the same manner as Comparative Example 1.

### [First reaction step]

Step (3A): A solution containing non-fat dry milk at 5% w/v, 1 × TBS, and 0.1% Tween (registered trademark) 20 was prepared.
Step (3B): Non-fluorescent melamine nanoparticles of which particle surfaces are bound with plural anti PD-L1 antibodies (E1L3N) as prepared in Preparation Example 5 were dispersed in the solution prepared in Step (3A) such that the mole number and concentration of the anti PD-L1 antibody are 10 times the mole number and concentration (mol/µL) of the primary antibody used in Comparative Example 1. Accordingly, a dispersion liquid was prepared in an amount of 100 µL. Then, the entire amount of the dispersion liquid was applied on top of "PD-L1 culture cells" and incubated overnight at 4°C.

Specifically, based on the information described in the following formula 1, which is obtained according to the method explained for determination of antibody binding density described in Preparation Example 4, mole number of antibody contained in 1 µL of the dispersion liquid of non-fluorescent nanoparticles that are bound with the primary antibody (see, Preparation Example 5) is calculated by the following formula 1, and based on the obtained information, use amount of the primary antibody was adjusted to have the same mole number and the same concentration (mol/µL) as the mole number of the primary antibody used in Comparative Example 1.

### (Formula 1)

Mole number/µL = "Concentration of dispersion liquid of non-fluorescent nanoparticles (particles/µL)" × "Average particle surface area (mm²/particle)" × "Antibody binding density (antibody mole number/mm²)" on surface of non-fluorescent nanoparticle

### [Result]

As a result of Example 4, the signal value compared to Comparative Example 1 was found to be 4, and the noise value (relative value) was found to be 3. S/N ratio (relative value) was 1.33. Furthermore, the quantification property was " ○". Result of Example 4 is shown in Table 3.

### [Example 5] (Primary antibody: monoclonal antibody directly bound to non-fluorescent nanoparticle, secondary antibody: free polyclonal antibody)

In Example 5, except that the first reaction step and the second reaction step of Comparative Example 1 are carried out as described below, other steps including the deparaffinization treatment step, the activation treatment step, the washing steps 1 to 3, the third reaction step, and the observation step were carried out in the same manner as Comparative Example 1.

### [First reaction step]

Step (3A): A solution containing non-fat dry milk at 5% w/v, 1 × TBS, and 0.1% Tween (registered trademark) 20 was prepared.
Step (3B): Non-fluorescent melamine nanoparticles of which particle surfaces are bound with plural anti PD-L1 antibodies (E1L3N) as prepared in Preparation Example 5 were dispersed in the solution prepared in Step (3A) such that the mole number and concentration of the anti PD-L1 antibody are 10 times the mole number and concentration (mol/µL) of the primary antibody used in Comparative Example 1. Accordingly, a dispersion liquid was prepared in an amount of 100 µL. Then, the entire amount of the dispersion liquid was applied on top of "PD-L1 culture cells" and incubated overnight at 4°C.

Specifically, use amount of the primary antibody was determined based on the formula 1 as it is described in Example 4.

### [Second reaction step]

Step (5A): By using the solution which has been prepared in Step (3A), the antibody solution of the biotin-modified free polyclonal antibody (secondary antibody) "Histofine (registered trademark)" (biotin labeled anti rabbit IgG antibody, animal species: goat, product number: 426011, 426012, NICHIREI BIOSCIENCES INC.) prepared in Preparation Example 6 was diluted 200 times, 100 µL of the resulting solution was applied on top of a sample slide (sample slide of "PD-L1 culture cells") obtained after Step (4A), and the reaction was allowed to occur for 30 minutes at room temperature.

### [Result]

As a result of Example 5, the signal value compared to Comparative Example 1 was found to be 8, and the noise value (relative value) was found to be 6. S/N ratio (relative value) was 1.33. Furthermore, the quantification property was "○". Result of Example 5 is shown in Table 3.

### [Example 6] (Primary antibody: polyclonal antibody directly bound to non-fluorescent nanoparticle, secondary antibody: free monoclonal antibody)

In Example 6, except that the first reaction step and the second reaction step of Comparative Example 1 are carried out as described below, other steps including the deparaffinization treatment step, the activation treatment step, the washing steps 1 to 3, the third reaction step, and the observation step were carried out in the same manner as Comparative Example 1.

### [First reaction step]

Step (3A): A solution containing non-fat dry milk at 5% w/v, 1 × TBS, and 0.1% Tween (registered trademark) 20 was prepared.
Step (3B): Non-fluorescent melamine nanoparticles of which particle surfaces are bound with polyclonal antibody (ab58810), which is anti PD-L1 antibody, as prepared in Preparation Example 4 were dispersed in the solution prepared in Step (3A) such that the mole number and concentration of the anti PD-L1 antibody are 10 times the mole number and concentration (mol/µL) of the primary antibody used in Comparative Example 1. Accordingly, a dispersion liquid was prepared in an amount of 100 µL. Then, the entire amount of the dispersion liquid was applied on top of "PD-L 1 culture cells" and incubated overnight at 4°C.

Specifically, use amount of the primary antibody was determined based on the formula 1 as it is described in Example 4.

### [Result]

As a result of Example 6, the signal value compared to Comparative Example 1 was found to be 8, and the noise value (relative value) was found to be 4. S/N ratio (relative value) was 2. Furthermore, the quantification property was "○". Result of Example 6 is shown in Table 3.

### [Example 7] (Primary antibody: polyclonal antibody directly bound to non-fluorescent nanoparticle, secondary antibody: free polyclonal antibody)

In Example 7, except that the first reaction step and the second reaction step of Comparative Example 1 are carried out as described below, other steps including the deparaffinization treatment step, the activation treatment step, the washing steps 1 to 3, the third reaction step, and the observation step were carried out in the same manner as Comparative Example 1.

### [First reaction step]

Step (3A): A solution containing non-fat dry milk at 5% w/v, 1 × TBS, and 0.1% Tween (registered trademark) 20 was prepared.
Step (3B): Non-fluorescent melamine nanoparticles of which particle surfaces are bound with polyclonal antibody (ab58810), which is anti PD-L1 antibody, as prepared in Preparation Example 4 were dispersed in the solution prepared in Step (3A) such that the mole number and concentration of the anti PD-L1 antibody are 10 times the mole number and concentration (mol/µL) of the primary antibody used in Comparative Example 1. Accordingly, a dispersion liquid was prepared in an amount of 100 µL. Then, the entire amount of the dispersion liquid was applied on top of "PD-L 1 culture cells" and incubated overnight at 4°C.

Specifically, use amount of the primary antibody was determined based on the formula 1 as it is described in Example 4.

### [Second reaction step]

Step (5A): By using the solution which has been prepared in Step (3A), the antibody solution of the biotin-modified free polyclonal antibody (secondary antibody) "Histofine (registered trademark)" (biotin labeled anti rabbit IgG antibody, animal species: goat, product number: 426011, 426012, NICHIREI BIOSCIENCES INC.) prepared in Preparation Example 6 was diluted 200 times, 100 µL of the resulting solution was applied on top of a sample slide obtained after Step (4A) (sample slide of "PD-L1 culture cells"), and the reaction was allowed to occur for 30 minutes at room temperature.

### [Result]

As a result of Example 7, the signal value compared to Comparative Example 1 was found to be 12, and the noise value (relative value) was found to be 8. S/N ratio (relative value) was 1.50. Furthermore, the quantification property was "○". Result of Example 7 is shown in Table 3.

### [Example 8] (Primary antibody: free monoclonal antibody, secondary antibody: monoclonal antibody directly bound to fluorescent nanoparticle)

In Example 8, except that the second reaction step of Comparative Example 1 is carried out as described below, other steps including the deparaffinization treatment step, the activation treatment step, the washing steps 1 to 3, the first reaction step, the third reaction step, and the observation step were carried out in the same manner as Comparative Example 1.

### [Second reaction step]

Step (5A): By using the solution which has been prepared in Step (3A) of Comparative Example 1, the fluorescent nanoparticles directly bound with monoclonal antibody "LO-RG-1" which has been prepared in Preparation Example 3 were diluted such that the second antibody has the mole number and concentration (mol/µL) that are 10 times of the secondary antibody used in Comparative Example 1. Accordingly, a dispersion liquid was prepared. Then, 100 µL of the dispersion liquid was applied on top of a sample slide (sample slide of "PD-L 1 culture cells") obtained from Step (4A) and the reaction was allowed to occur for 30 minutes at room temperature.

### [Result]

As a result of Example 8, the signal value compared to Comparative Example 1 was found to be 1, and the noise value (relative value) was found to be 0.8. S/N ratio (relative value) was 1.25. Furthermore, the quantification property was "○". Result of Example 8 is shown in Table 3.

### [Example 9] (Primary antibody: free monoclonal antibody, polyclonal antibody directly bound to fluorescent nanoparticle)

In Example 9, except that the second reaction step of Comparative Example 1 is carried out as described below, other steps including the deparaffinization treatment step, the activation treatment step, the washing steps 1 to 3, the first reaction step, the third reaction step, and the observation step were carried out in the same manner as Comparative Example 1.

### [Second reaction step]

Step (5A): By using the solution which has been prepared in Step (3A) of Comparative Example 1, the fluorescent nanoparticles directly bound with polyclonal antibody (secondary antibody)" Histofine" which have been prepared in Preparation Example 4 were diluted such that the second antibody has the mole number and concentration (mol/µL) that are 10 times of the secondary antibody used in Comparative Example 1. Accordingly, a dispersion liquid was prepared. Then, 100 µL of the dispersion liquid was applied on top of sample slide (sample slide of "PD-L 1 culture cells") obtained from Step (4A) and the reaction was allowed to occur for 30 minutes at room temperature.

### [Result]

As a result of Example 9, the signal value compared to Comparative Example 1 was found to be 2, and the noise value (relative value) was found to be 1.5. S/N ratio (relative value) was 1.33. Furthermore, the quantification property was "○". Result of Example 9 is shown in Table 3.

### [Example 10] (Primary antibody: free polyclonal antibody, monoclonal antibody directly bound to fluorescent nanoparticle)

In Example 10, except that the first reaction step and the second reaction step of Comparative Example 1 are carried out as described below, other steps including the deparaffinization treatment step, the activation treatment step, the washing steps 1 to 3, the third reaction step, and the observation step were carried out in the same manner as Comparative Example 1.

### [First reaction step]

Step (3A): A solution containing non-fat dry milk at 5% w/v, 1 × TBS, and 0.1% Tween (registered trademark) 20 was prepared.
Step (3B): By using the solution which has been prepared in Step (3A), "Anti-PD-L1 antibody (ab58810)" (animal species: rabbit, Abcam), which is a polyclonal antibody capable of binding to human PD-L1, was diluted 200 times, and 100 µL of the resulting antibody solution was applied on top of a sample slide of PD-L1 culture cells and incubated overnight at 4°C.

Furthermore, the primary antibody was used after it has been adjusted to have the same mole number as the mole number of the primary antibody used in Comparative Example 1.

### [Second reaction step]

Step (5A): By using the solution which has been prepared in Step (3A) of Comparative Example 1, the fluorescent nanoparticles directly bound with monoclonal antibody "LO-RG-1" which has been prepared in Preparation Example 3 were diluted such that the second antibody has the mole number and concentration (mol/µL) that are 10 times of the secondary antibody used in Comparative Example 1. Accordingly, a dispersion liquid was prepared. Then, 100 µL of the dispersion liquid was applied on top of a sample slide (sample slide of "PD-L 1 culture cells") obtained from Step (4A) and the reaction was allowed to occur for 30 minutes at room temperature.

### [Result]

As a result of Example 10, the signal value compared to Comparative Example 1 was found to be 4, and the noise value (relative value) was found to be 2.5. S/N ratio (relative value) was 1.60. Furthermore, the quantification property was "○". Result of Example 10 is shown in Table 3.

### [Example 11] (Primary antibody: free polyclonal antibody, monoclonal antibody directly bound to fluorescent nanoparticle)

In Example 11, except that the first reaction step and the second reaction step of Comparative Example 1 are carried out as described below, other steps including the deparaffinization treatment step, the activation treatment step, the washing steps 1 to 3, the third reaction step, and the observation step were carried out in the same manner as Comparative Example 1.

### [First reaction step]

Step (3A): A solution containing non-fat dry milk at 5% w/v, 1 × TBS, and 0.1% Tween (registered trademark) 20 was prepared.
Step (3B): By using the solution which has been prepared in Step (3A), "Anti-PD-Ll antibody (ab58810)" (animal species: rabbit, Abcam), which is a polyclonal antibody capable of binding to human PD-L1, was diluted 200 times, and 100 µL of the resulting antibody solution was applied on top of a sample slide of PD-L1 culture cells and incubated overnight at 4°C.

Furthermore, the primary antibody was used after it has been adjusted to have the same mole number as the mole number of the primary antibody used in Comparative Example 1.

### [Second reaction step]

Step (5A): By using the solution which has been prepared in Step (3A) of Comparative Example 1, the fluorescent nanoparticles directly bound with polyclonal antibody (secondary antibody) "Histofine" which have been prepared in Preparation Example 4 were diluted such that the second antibody has the mole number and concentration (mol/µL) that are 10 times of the secondary antibody used in Comparative Example 1. Accordingly, a dispersion liquid was prepared. Then, 100 µL of the dispersion liquid was applied on top of a sample slide (sample slide of "PD-L 1 culture cells") obtained from Step (4A) and the reaction was allowed to occur for 30 minutes at room temperature.

### [Result]

As a result of Example 11, the signal value compared to Comparative Example 1 was found to be 8, and the noise value (relative value) was found to be 4.5. S/N ratio (relative value) was 1.78. Furthermore, the quantification property was "○". Result of Example 11 is shown in Table 3.

### [Example 12] (Primary antibody: monoclonal antibody directly bound to non-fluorescent nanoparticle, monoclonal antibody directly bound to fluorescent nanoparticle)

In Example 12, except that the first reaction step and the second reaction step of Comparative Example 1 are carried out as described below, other steps including the deparaffinization treatment step, the activation treatment step, the washing steps 1 to 3, the third reaction step, and the observation step were carried out in the same manner as Comparative Example 1.

### [First reaction step]

Step (3A): A solution containing non-fat dry milk at 5% w/v, 1 × TBS, and 0.1% Tween (registered trademark) 20 was prepared.
Step (3B): By using the solution which has been prepared in Step (3A), non-fluorescent melamine nanoparticles (non-fluorescent nanoparticles) of which particle surfaces are bound with plural anti PD-L1 antibodies (E1L3N) as prepared in Preparation Example 5 were dispersed such that the mole number and concentration of the anti PD-L1 antibody are 10 times the mole number and concentration (mol/µL) of the primary antibody used in Comparative Example 1. Accordingly, a dispersion liquid was prepared in an amount of 100 µL. Then, the entire amount of the dispersion liquid was applied on top of "PD-L 1 culture cells" and incubated overnight at 4°C.

Specifically, use amount of the primary antibody was determined based on the formula 1 as it is described in Example 4.

### [Second reaction step]

Step (5A): By using the solution which has been prepared in Step (3A) of Comparative Example 1, the fluorescent nanoparticles directly bound with monoclonal antibody "LO-RG-1" which have been prepared in Preparation Example 3 were diluted such that the second antibody has the mole number and concentration (mol/µL) that are 10 times of the secondary antibody used in Comparative Example 1. Accordingly, a dispersion liquid was prepared. Then, 100 µL of the dispersion liquid was applied on top of a sample slide (sample slide of "PD-L 1 culture cells") obtained from Step (4A) and the reaction was allowed to occur for 30 minutes at room temperature.

### [Result]

As a result of Example 12, the signal value compared to Comparative Example 1 was found to be 8, and the noise value (relative value) was found to be 5.5. S/N ratio (relative value) was 1.45. Furthermore, the quantification property was "○". Result of Example 12 is shown in Table 3.

### [Example 13] (Primary antibody: monoclonal antibody directly bound to non-fluorescent nanoparticle, polyclonal antibody directly bound to fluorescent nanoparticle)

In Example 13, except that the first reaction step and the second reaction step of Comparative Example 1 are carried out as described below, other steps including the deparaffinization treatment step, the activation treatment step, the washing steps 1 to 3, the third reaction step, and the observation step were carried out in the same manner as Comparative Example 1.

### [First reaction step]

Step (3A): A solution containing non-fat dry milk at 5% w/v, 1 × TBS, and 0.1% Tween (registered trademark) 20 was prepared.
Step (3B): By using the solution which has been prepared in Step (3A), non-fluorescent melamine nanoparticles (non-fluorescent nanoparticles) of which particle surfaces are bound with plural anti PD-L1 antibodies (E1L3N) prepared in Preparation Example 5 were dispersed such that the mole number and concentration of the anti PD-L1 antibody are 10 times the mole number and concentration (mol/µL) of the primary antibody used in Comparative Example 1. Accordingly, a dispersion liquid was prepared in an amount of 100 µL. Then, the entire amount of the dispersion liquid was applied on top of "PD-L 1 culture cells" and incubated overnight at 4°C.

Specifically, use amount of the primary antibody was determined based on the formula 1 as it is described in Example 4.

### [Second reaction step]

Step (5A): By using the solution which has been prepared in Step (3A) of Comparative Example 1, the fluorescent nanoparticles directly bound with polyclonal antibody (secondary antibody) "Histofine", which has been prepared in Preparation Example 4, were diluted such that the second antibody has the mole number and concentration (mol/µL) that are 10 times of the secondary antibody used in Comparative Example 1. Accordingly, a dispersion liquid was prepared. Then, 100 µL of the dispersion liquid was applied on top of a sample slide (sample slide of "PD-L 1 culture cells") obtained from Step (4A) and the reaction was allowed to occur for 30 minutes at room temperature.

### [Result]

As a result of Example 13, the signal value compared to Comparative Example 1 was found to be 12, and the noise value (relative value) was found to be 6.5. S/N ratio (relative value) was 1.85. Furthermore, the quantification property was "○". Result of Example 13 is shown in Table 3.

### [Example 14] (Primary antibody: polyclonal antibody directly bound to non-fluorescent nanoparticle, monoclonal antibody directly bound to fluorescent nanoparticle)

In Example 14, except that the first reaction step and the second reaction step of Comparative Example 1 are carried out as described below, other steps including the deparaffinization treatment step, the activation treatment step, the washing steps 1 to 3, the third reaction step, and the observation step were carried out in the same manner as Comparative Example 1.

### [First reaction step]

Step (3A): A solution containing non-fat dry milk at 5% w/v, 1 × TBS, and 0.1% Tween (registered trademark) 20 was prepared.
Step (3B): Non-fluorescent melamine nanoparticles of which particle surfaces are bound with polyclonal antibody (ab58810), which is anti PD-L1 antibody, as prepared in Preparation Example 4, were dispersed in the solution prepared in Step (3A) such that the mole number and concentration of the anti PD-L1 antibody are 10 times the mole number and concentration (mol/µL) of the primary antibody used in Comparative Example 1. Accordingly, a dispersion liquid was prepared in an amount of 100 µL. Then, the entire amount of the dispersion liquid was applied on top of "PD-L 1 culture cells" and incubated overnight at 4°C.

Specifically, use amount of the primary antibody was determined based on the formula 1 as it is described in Example 4.

### [Second reaction step]

Step (5A): By using the solution which has been prepared in Step (3A) of Comparative Example 1, the fluorescent nanoparticles directly bound with monoclonal antibody "LO-RG-1" which have been prepared in Preparation Example 3 were diluted such the second antibody has the mole number and concentration (mol/µL) that are 10 times of the secondary antibody used in Comparative Example 1. Accordingly, a dispersion liquid was prepared. Then, 100 µL of the dispersion liquid was applied on top of a sample slide (sample slide of "PD-L 1 culture cells") obtained from Step (4A) and the reaction was allowed to occur for 30 minutes at room temperature.

### [Result]

As a result of Example 14, the signal value compared to Comparative Example 1 was found to be 16, and the noise value (relative value) was found to be 10. S/N ratio (relative value) was 1.60. Furthermore, the quantification property was "○". Result of Example 14 is shown in Table 3.

### [Example 15] (Primary antibody: polyclonal antibody directly bound to non-fluorescent nanoparticle, polyclonal antibody directly bound to fluorescent nanoparticle)

In Example 15, except that the first reaction step and the second reaction step of Comparative Example 1 are carried out as described below, other steps including the deparaffinization treatment step, the activation treatment step, the washing steps 1 to 3, the third reaction step, and the observation step were carried out in the same manner as Comparative Example 1.

### [First reaction step]

Step (3A): A solution containing non-fat dry milk at 5% w/v, 1 × TBS, and 0.1% Tween (registered trademark) 20 was prepared.
Step (3B): Non-fluorescent melamine nanoparticles of which particle surfaces are bound with polyclonal antibody (ab58810), which is anti PD-L1 antibody, as prepared in Preparation Example 4 were dispersed in the solution prepared in Step (3A) such that the mole number and concentration of the anti PD-L1 antibody are 10 times the mole number and concentration (mol/µL) of the primary antibody used in Comparative Example 1. Accordingly, a dispersion liquid was prepared in an amount of 100 µL. Then, the entire amount of the dispersion liquid was applied on top of "PD-L 1 culture cells" and incubated overnight at 4°C.

Specifically, use amount of the primary antibody was determined based on the formula 1 as it is described in Example 4.

### [Second reaction step]

Step (5A): By using the solution which has been prepared in Step (3A) of Comparative Example 1, the fluorescent nanoparticles directly bound with polyclonal antibody (secondary antibody) "Histofine" which have been prepared in Preparation Example 4 were diluted such that the second antibody has the mole number and concentration (mol/µL) that are 10 times of the secondary antibody used in Comparative Example 1. Accordingly, a dispersion liquid was prepared. Then, 100 µL of the dispersion liquid was applied on top of a sample slide (sample slide of "PD-L 1 culture cells") obtained from Step (4A) and the reaction was allowed to occur for 30 minutes at room temperature.

### [Results]

As a result of Example 15, the signal value compared to Comparative Example 1 was found to be 24, and the noise value (relative value) was found to be 16. S/N ratio (relative value) was 1.50. Furthermore, the quantification property was "○". Result of Example 15 is shown in Table 3.

**[Table 3]**

| | Primary antibody | Secondary antibody | Signal (relative value) | Noise (relative value) | S/N ratio (relative value) | Quantification property ** |
|---|---|---|---|---|---|---|
| Comparative Example 1 | Monoclonal (E1L3N) Antigen species: human, animal species: rabbit | Monoclonal (LO-RG-1) Antigen species: rabbit, animal species: rat | 1 | 1 | 1.00 | ○ |
| Example 1 | Monoclonal (E1L3N) Antigen species: human, animal species: rabbit | Polyclonal (Histofine) Antigen species: rabbit, animal species: goat | 2 | 1.5 | 1.33 | ○ |
| Example 2 | Polyclonal (ab58810) Antigen species: human, animal species: rabbit | Monoclonal (LO-RG-1) Antigen species: rabbit, animal species: rat | 2 | 1 | 2.00 | ○ |
| Example 3 | Polyclonal antibody (ab58810) Antigen species: human, animal species: rabbit | Polyclonal (Histofine) Antigen species: rabbit, animal species: goat | 4 | 3 | 1.33 | ○ |
| Example 4 | Non-fluorescent nanoparticle directly bound with monoclonal antibody (E1L3N) Antigen species: human, animal species: rabbit | Monoclonal (LO-RG-1) Antigen species: rabbit, animal species: rat | 4 | 3 | 1.33 | ○ |
| Example 5 | Non-fluorescent nanoparticle directly bound with monoclonal antibody (E1L3N) Antigen species: human, animal species: rabbit | Polyclonal (Histofine) Antigen species: rabbit, animal species: goat | 8 | 6 | 1.33 | ○ |
| Example 6 | Non-fluorescent nanoparticle directly bound with polyclonal antibody (ab58810) Antigen species: human, animal species: rabbit | Monoclonal (LO-RG-1) Antigen species: rabbit, animal species: rat | 8 | 4 | 2.00 | ○ |
| Example 7 | Non-fluorescent nanoparticle directly bound with polyclonal antibody (ab58810) Antigen species: human, animal species: rabbit | Polyclonal (Histofine) Antigen species: rabbit, animal species: goat | 12 | 8 | 1.50 | ○ |
| Example 8 | Monoclonal (E1L3N) Antigen species: human, animal species: rabbit | Fluorescent nanoparticle directly bound with monoclonal (LO-RG-1) Antigen species: rabbit, animal species: rat | 1 | 0.8 | 1.25 | ○ |
| Example 9 | Monoclonal (E1L3N) Antigen species: human, animal species: rabbit | Fluorescent nanoparticle directly bound with polyclonal (Histofine) Antigen species: rabbit, animal species: goat | 2 | 1.5 | 1.33 | ○ |
| Example 10 | Polyclonal antibody (ab58810) Antigen species: human, animal species: rabbit | Fluorescent nanoparticle directly bound with monoclonal (LO-RG-1) Antigen species: rabbit, animal species: rat | 4 | 2.5 | 1.60 | ○ |
| Example 11 | Polyclonal antibody (ab58810) Antigen species: human, animal species: rabbit | Fluorescent nanoparticle directly bound with polyclonal (Histofine) Antigen species: rabbit, animal species: goat | 8 | 4.5 | 1.78 | ○ |
| Example 12 | Non-fluorescent nanoparticle directly bound with monoclonal antibody (E1L3N) Antigen species: human, animal species: rabbit | Fluorescent nanoparticle directly bound with monoclonal (LO-RG-1) Antigen species: rabbit, animal species: rat | 8 | 5.5 | 1.45 | ○ |
| Example 13 | Non-fluorescent nanoparticle directly bound with monoclonal antibody (E1L3N) Antigen species: human, animal species: rabbit | Fluorescent nanoparticle directly bound with polyclonal (Histofine) Antigen species: rabbit, animal species: goat | 12 | 6.5 | 1.85 | ○ |
| Example 14 | Non-fluorescent nanoparticle directly bound with polyclonal antibody (ab58810) Antigen species: human, animal species: rabbit | Fluorescent nanoparticle directly bound with monoclonal (LO-RG-1) Antigen species: rabbit, animal species: rat | 16 | 10 | 1.6 | ○ |
| Example 15 | Non-fluorescent nanoparticle directly bound with polyclonal antibody (ab58810) Antigen species: human, animal species: rabbit | Fluorescent nanoparticle directly bound with polyclonal (Histofine) Antigen species: rabbit, animal species: goat | 24 | 16 | 1.5 | ○ |

| | | | | | | |
|---|---|---|---|---|---|---|
| ** Evaluation of particle number | | | | | | |

### [Discussions]

In all of Examples 1 to 15, the S/N ratio was enhanced compared to Comparative Example 1.

Namely, according to the constitution in which one antigen is bound with 2 or more secondary antibodies which have been fluorescence labeled (or, to be subsequently fluorescence labeled) with a fluorescent nanoparticle, enhanced S/N ratio is obtained compared to the fluorescent immunostaining method of Comparative Example 1 in which one antigen is bound with one monoclonal antibody (secondary antibody).

### (Case in which free primary antibody and secondary antibody are used)

Based on the result of Table 3, in case of using a free polyclonal antibody, it is preferable from the viewpoint of the S/N ratio to have a fluorescent immunostaining method in which a free polyclonal antibody is used as a primary antibody and, simultaneously, a free monoclonal antibody is used as a secondary antibody so that plural secondary antibodies can be fixed to one molecule of the antigen. Furthermore, from the viewpoint of signal enhancement, it is preferable to have a fluorescent immunostaining method in which a free polyclonal antibody is used for both the primary antibody and the secondary antibody so that plural secondary antibodies can be fixed to one molecule of the antigen.

### (Case in which primary antibody directly bound to non-fluorescent nanoparticle and free secondary antibody are used)

Based on the result of Table 3, in case of using a primary antibody directly bound to a non-fluorescent nanoparticle and a free secondary antibody, it is preferable from the viewpoint of the S/N ratio to have a fluorescent immunostaining method in which a polyclonal antibody bound in large number on a surface of a non-fluorescent nanoparticle is used as a primary antibody, and, simultaneously, a free monoclonal antibody is used as a secondary antibody so that plural secondary antibodies can be fixed to one molecule of the antigen.

Furthermore, from the viewpoint of signal enhancement, it is preferable to have a fluorescent immunostaining method in which a polyclonal antibody bound in large number on a surface of a non-fluorescent nanoparticle is used as a primary antibody, and, simultaneously, a free polyclonal antibody is used as a secondary antibody so that plural secondary antibodies can be fixed to one molecule of the antigen.

### (Case in which free primary antibody and secondary antibody directly bound to fluorescent nanoparticle are used)

Based on the result of Table 3, in case of using a free primary antibody and a secondary antibody directly bound to a fluorescent nanoparticle, from the viewpoint of both the S/N ratio and signal enhancement, it is preferable to have a fluorescent immunostaining method in which a free polyclonal antibody is used as a primary antibody and, simultaneously, a polyclonal antibody directly bound to a fluorescent nanoparticle is used as a secondary antibody so that plural secondary antibodies can be fixed to one molecule of the antigen.

### (Case in which primary antibody directly bound to non-fluorescent nanoparticle and secondary antibody directly bound to fluorescent nanoparticle are used)

Based on the result of Table 3, in case of using a primary antibody directly bound to a non-fluorescent nanoparticle and a secondary antibody directly bound to a fluorescent nanoparticle, it is preferable from the viewpoint of the S/N ratio to have a fluorescent immunostaining method in which a monoclonal antibody bound in large number on a surface of a non-fluorescent nanoparticle is used as a primary antibody, and, simultaneously, a polyclonal antibody directly bound to a fluorescent nanoparticle is used as a secondary antibody so that plural secondary antibodies can be fixed to one molecule of the antigen.

Furthermore, from the viewpoint of signal enhancement, it is preferable to have a fluorescent immunostaining method in which a polyclonal antibody bound in large number on a surface of a non-fluorescent nanoparticle is used as a primary antibody, and, simultaneously, a polyclonal antibody bound in large number on a surface of a fluorescent nanoparticle is used as a secondary antibody so that plural secondary antibodies can be fixed to one molecule of the antigen.

In the above, the present invention is explained based on embodiments and examples thereof. However, the present invention is not limited to them and design modifications can be made as long as they do not deviate from the gist of the present invention described in claims of the present invention.

### Reference Signs List

- 1a, 1b: Primary antibody
- 2a to 2f: Secondary antibody
- 3: Biotin (first binding group material)
- 4: Streptavidin (second binding group material)
- 5: Fluorescent nanoparticle
- P: Antigen
- ep1: First epitope
- ep2: Second epitope

## Claims

1. A fluorescent immunostaining method comprising: a first reaction step of specifically binding and fixing a primary antibody to an antigen as a staining subject; a second reaction step of specifically binding a secondary antibody to the bound and fixed primary antibody; and a third reaction step of fluorescence labeling the secondary antibody with a fluorescent nanoparticle,
wherein a plurality of secondary antibodies are fixed to one molecule of the antigen by way of the primary antibody.

2. The fluorescent immunostaining method according to claim 1, wherein the primary antibody is a free primary antibody and the secondary antibody is a free secondary antibody.

3. The fluorescent immunostaining method according to claim 1, wherein the primary antibody is a primary antibody fixed on a surface of a non-fluorescent nanoparticle not containing fluorescent material and the secondary antibody is a free secondary antibody.

4. The fluorescent immunostaining method according to claim 1, wherein the primary antibody is a free primary antibody and the secondary antibody is a secondary antibody fixed on a surface of a fluorescent nanoparticle.

5. The fluorescent immunostaining method according to claim 1, wherein the primary antibody is a primary antibody fixed on a surface of a non-fluorescent nanoparticle and the secondary antibody is a secondary antibody fixed on a surface of a fluorescent nanoparticle.

6. The fluorescent immunostaining method according to any one of claims 1 to 5, wherein the primary antibody is a polyclonal antibody and the secondary antibody is a monoclonal antibody.

7. The fluorescent immunostaining method according to any one of claims 1 to 5, wherein the primary antibody is a monoclonal antibody and the secondary antibody is a polyclonal antibody.

8. The fluorescent immunostaining method according to any one of claims 1 to 5, wherein the primary antibody is a polyclonal antibody and the secondary antibody is a polyclonal antibody.

9. The fluorescent immunostaining method according to any one of claims 1 and 3 to 5, wherein the primary antibody is a monoclonal antibody and the secondary antibody is a monoclonal antibody.

10. The fluorescent immunostaining method according to claim 1, wherein
a second binding group material is added to the secondary antibody, a first binding group material is added to the fluorescent nanoparticle, and
after the second reaction step, according to binding between the secondary antibody and fluorescent nanoparticle by way of specific binding between the first binding group material and second binding group material, the fluorescence labeling is achieved.

11. The fluorescent immunostaining method according to claim 10, wherein combination of the first binding group material and second binding group material is biotin/avidin, or hapten/anti hapten antibody.
